(19) Europäisches Patentamt
European Patent Office
Office européen des brevets

(11) Veröffentlichungsnummer: **0 166 422**
**A1**

(12) # EUROPÄISCHE PATENTANMELDUNG

(21) Anmeldenummer: **85107835.2**

(22) Anmeldetag: **25.06.85**

(51) Int. Cl.⁴: **C 07 D 211/90**, C 07 D 401/04, C 07 D 417/06, C 07 D 275/02, C 07 C 143/76, A 61 K 31/455

(30) Priorität: **27.06.84 CH 3097/84**

(43) Veröffentlichungstag der Anmeldung: **02.01.86 Patentblatt 86/1**

(84) Benannte Vertragsstaaten: **AT BE CH DE FR GB IT LI LU NL SE**

(71) Anmelder: **F. HOFFMANN-LA ROCHE & CO. Aktiengesellschaft, CH-4002 Basel (CH)**

(72) Erfinder: **Jaunin, Roland, Prof. Dr., Lehenmattstrasse 308, CH-4052 Basel (CH)**
Erfinder: **Ramuz, Henri, Prof. Dr., Rheinparkstrasse 3, CH-4127 Birsfelden (CH)**

(74) Vertreter: **Lederer, Franz, Dr. et al, Patentanwälte Dr. Franz Lederer Dipl.-Ing. Reiner F. Meyer-Roxlau Lucile-Grahn-Strasse 22, D-8000 München 80 (DE)**

(54) **Dihydropyridinderivate, Verfahren und Zwischenprodukte zu deren Herstellung sowie diese enthaltende Arzneimittel.**

(57) Es wurde gefunden, dass die neuen Dihydropyridinderivate der Formel

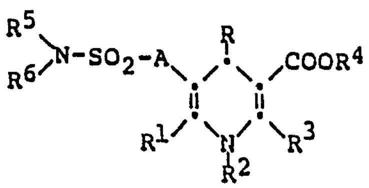

I

worin die Symbole A, R und $R^1$ bis $R^7$ die in Anspruch 1 angegebene Bedeutung besitzen, eine ausgeprägte Calcium-antagonistische Wirkung besitzen und demnach als Arzneimittel verwendet werden können, insbesondere für die Bekämpfung bzw. Verhütung von Angina pectoris, Ischämie, Bluthochdruck und/oder Migräne. Die Verbindungen der Formel I können durch Umsetzen eines ebenfalls neuen Enamins der Formel

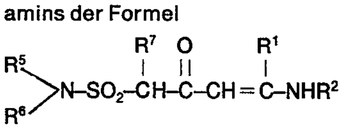

II

mit einem Yliden der Formel

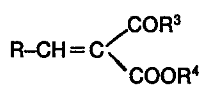

III

und gegebenenfalls nachfolgender Behandlung mit Natriumborhydrid hergestellt werden.

EP 0 166 422 A1

F. Hoffmann-La Roche & Co. Aktiengesellschaft, Basel, Schweiz

RAN 4029/1

# Dihydropyridinderivate, Verfahren und Zwischenprodukte zu deren Herstellung sowie diese enthaltende Arzneimittel

Die vorliegende Erfindung betrifft Dihydropyridinderivate. Im Speziellen betrifft sie Dihydropyridinderivate der allgemeinen Formel

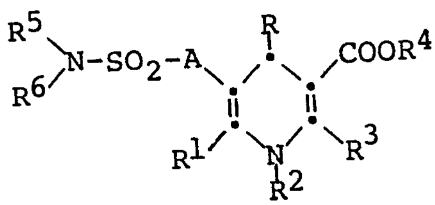

I

worin A die Gruppe $-CH(R^7)-CO-$ oder $-CH=CH-$ (E), R Aryl oder einen heterocyclischen Rest mit bis zu drei Heteroatomen, wobei als Heteroatome Sauerstoff, Stickstoff oder Schwefel in Frage kommen, $R^1$ Wasserstoff oder Methyl, $R^2$ Wasserstoff oder $C_1$-$C_4$-Alkyl, $R^3$ $C_1$-$C_4$-Alkyl oder $C_1$-$C_4$-Alkanoyloxymethyl, $R^4$ $C_1$-$C_6$-Alkyl, $C_3$-$C_6$-Alkenyl, $C_3$-$C_6$-Alkinyl, $C_3$-$C_6$-Cycloalkyl, $C_3$-$C_6$-Cycloalkyl-$C_1$-$C_6$-alkyl, Cyan-$C_2$-$C_6$-alkyl, Halogen-$C_2$-$C_6$-alkyl, Hydroxy-$C_2$-$C_6$-alkyl, ω,ω,ω-Trifluor-$C_1$-$C_6$-alkyl, $C_1$-$C_6$-Alkoxy-$C_1$-$C_6$-alkyl, $C_1$-$C_6$-Alkoxy-$C_1$-$C_6$-alkoxy-$C_1$-$C_6$-alkyl, $C_1$-$C_4$-Alkanoyloxy-$C_1$-$C_6$-alkyl, $C_3$-$C_6$-Alkenyloxy-$C_1$-$C_6$-alkyl, $C_1$-$C_6$-Alkylthio-$C_1$-

Kbr/30.4.85

$C_6$-alkyl, Benzyloxy-$C_1$-$C_6$-alkyl, gegebenenfalls durch Halogen, Cyan, Di-$C_1$-$C_6$-alkylamino, $C_1$-$C_6$-Alkoxy, $C_1$-$C_6$-Alkyl, Trifluormethyl oder Nitro substituiertes Phenyl oder gegebenenfalls durch Halogen, Cyan, Di-$C_1$-$C_6$-alkylamino, $C_1$-$C_6$-Alkoxy, $C_1$-$C_6$-Alkyl, Trifluormethyl oder Nitro substituiertes Phenyl-$C_1$-$C_6$-alkyl, $R^5$ $C_1$-$C_6$-Alkyl oder $C_3$-$C_6$-Cycloalkyl, $R^6$ Wasserstoff, $C_1$-$C_6$-Alkyl, $C_3$-$C_6$-Alkenyl, $C_3$-$C_6$-Alkinyl, $C_3$-$C_6$-Cycloalkyl, $C_3$-$C_6$-Cycloalkyl-$C_1$-$C_6$-alkyl oder gegebenenfalls durch $C_1$-$C_6$-Alkyl, $C_1$-$C_6$-Alkoxy oder Halogen substituiertes Phenyl-$C_1$-$C_6$-alkyl und $R^7$ Wasserstoff, $C_1$-$C_6$-Alkyl oder gegebenenfalls durch $C_1$-$C_6$-Alkyl, $C_1$-$C_6$-Alkoxy oder Halogen substituiertes Phenyl-$C_1$-$C_6$-alkyl oder $R^6$ und $R^7$ zusammen eine $-(CH_2)_n-$-Gruppe, worin n die Zahl 2 oder 3 bedeutet, bedeuten, in Form von Isomeren, Isomerengemischen, Racematen und optischen Antipoden.

Diese Verbindungen sind neu und zeichnen sich durch wertvolle pharmakodynamische Eigenschaften aus.

Gegenstand der vorliegenden Erfindung sind Verbindungen der allgemeinen Formel I als solche und zur Anwendung als therapeutische Wirkstoffe, die Herstellung dieser Verbindungen, Zwischenprodukte für die Herstellung dieser Verbindungen, ferner diese enthaltende Arzneimittel und die Herstellung solcher Arzneimittel, sowie die Verwendung von Verbindungen der allgemeinen Formel I bei der Bekämpfung bzw. Verhütung von Krankheiten bzw. bei der Verbesserung der Gesundheit, insbesondere bei der Bekämpfung bzw. Verhütung von Angina pectoris, Ischämie, Bluthochdruck und Migräne.

Der in der vorliegenden Beschreibung verwendete Ausdruck "Alkyl" - allein oder in Kombination - bedeutet geradkettige und verzweigte, gesättigte Kohlenwasserstoffreste mit der jeweils angegebenen Anzahl Kohlenstoffatome, wie Methyl, Aethyl, n-Propyl, Isopropyl, n-Butyl, Isobutyl, sec.-Butyl, tert.-Butyl und dergleichen. Der Ausdruck "Alkoxy" bedeutet Alkyläthergruppen, worin der Ausdruck "Alkyl" die obige Bedeutung hat. Der Ausdruck "$C_3$-$C_6$--Alkenyl" umfasst geradkettige und verzweigte Kohlenwasserstoffgruppen mit 3-6 Kohlenstoffatomen, worin mindestens eine Kohlenstoff-Kohlenstoff-Bindung ungesättigt ist, wie Allyl, Butenyl und dergleichen. Der Ausdruck "$C_3$-$C_6$--Alkenyloxy" bedeutet Alkenyläthergruppen, worin der Ausdruck "$C_3$-$C_6$-Alkenyl" die obige Bedeutung hat. Der Ausdruck "$C_3$-$C_6$-Alkinyl" bezieht sich auf geradkettige und verzweigte Kohlenwasserstoffgruppen mit 3-6 Kohlenstoffatomen, worin mindestens eine Kohlenstoff-Kohlenstoff-Dreifachbindung vorhanden ist, wie Propargyl und dergleichen. Der Ausdruck "$C_3$-$C_6$-Cycloalkyl" bezieht sich auf cyclische, gesättigte Kohlenwasserstoffreste mit 3-6 Kohlenstoffatomen, wie Cyclopropyl, Cyclohexyl und dergleichen. Der Ausdruck "$C_1$-$C_4$-Alkanoyloxy" bezieht sich auf den Acyloxyrest einer Alkancarbonsäure mit 1-4 Kohlenstoffatomen, wie Formyloxy, Acetoxy, Propionyloxy, Butyryloxy und dergleichen. Der Ausdruck "Halogen" umfasst die vier Halogenatome Fluor, Chlor, Brom und Jod. Der Ausdruck "Aryl" umfasst einen gegebenenfalls durch Phenyl, $C_1$-$C_6$-Alkyl, $C_3$-$C_6$-Alkenyl, $C_3$-$C_6$-Alkinyl, $C_1$-$C_6$-Alkoxy, Halogen, Trifluormethyl, Trifluormethoxy, Difluormethoxy, Trifluormethylthio, Difluormethylthio, Nitro, Cyan, Azido, $C_1$-$C_6$-Alkoxycarbonyl, Aminocarbonyl, Aminosulfonyl, $C_1$-$C_6$-Alkylthio, $C_1$-$C_6$-Alkylsulfonyl oder $C_1$--$C_6$-Alkanoyl mono-, di- oder tri-substituierten oder durch $C_3$-$C_5$-Alkylen oder Dioxy-$C_1$-$C_2$-alkylen disubstituierten mono- oder bicyclischen aromatischen Kohlenwasserstoffrest mit bis zu 10 Kohlenstoffatomen im aromatischen Ringgerüst, wie Chlorphenyl, Tolyl, α,α,α-Trifluortolyl, Dichlorphenyl, Chlornitrophenyl, Naphthyl und dergleichen. Der Ausdruck "heterocyclischer Rest" umfasst 5- und 6-gliedrige mono- und bicyclische, gegebenenfalls

durch Phenyl, $C_1$-$C_6$-Alkyl, $C_3$-$C_6$-Alkenyl, $C_3$--$C_6$-Alkinyl, $C_1$-$C_6$-Alkoxy, Halogen, Trifluormethyl, Trifluormethoxy, Difluormethoxy, Trifluormethylthio, Difluormethylthio, Nitro, Cyan, Azido, $C_1$-$C_6$-Alkoxycarbonyl, Aminocarbonyl, Aminosulfonyl, $C_1$-$C_6$-Alkylthio, $C_1$-$C_6$-Alkylsulfonyl oder $C_1$-$C_6$-Alkanoyl mono-, di- oder tri-substituierte oder durch $C_3$-$C_5$--Alkylen oder Dioxy-$C_1$-$C_2$-alkylen disubstituierte Heterocyclen, wie Thienyl, Furyl, Pyrryl, Pyrazolyl, Imidazolyl, Oxazolyl, Isoxazolyl, Thiazolyl, Isothiazolyl, Pyridyl, N-Oxydopyridyl, Pyridazinyl, Pyrimidyl, Pyrazinyl, Chinolyl, Isochinolyl, Indolyl, Benzimidazolyl, Chinazolyl, Chinoxalyl, (2,1,3-Benzoxadiazol)-4-yl, (2,1,3-Benzothiadiazol)-4-yl, Benzofuranyl, Benzothienyl, Benzoxazolyl, Benzisoxazolyl, Benzothiazolyl, Benzisothiazolyl, Indazolyl und dergleichen. Der Ausdruck "$C_1$-$C_6$-Alkanoyl" bedeutet den Acylrest einer Alkancarbonsäure mit 1-6 Kohlenstoffatomen, wie Formyl, Acetyl, Propionyl, Butyryl und dergleichen. Der Ausdruck "Abgangsgruppe" bedeutet bekannte Gruppen wie Halogen, vorzugsweise Jod oder Brom, Arylsulfonyloxy, wie etwa Tosyloxy oder Brombenzolsulfonyloxy, oder Alkylsulfonyloxy, wie etwa Mesyloxy.

Eine besondere Klasse von Verbindungen der Formel I sind solche, worin $R^4$ $C_1$-$C_6$-Alkyl, Cyan-$C_2$-$C_6$--alkyl, Halogen-$C_2$-$C_6$-alkyl, Hydroxy-$C_2$-$C_6$-alkyl, $\omega,\omega,\omega$-Trifluor-$C_1$-$C_6$-alkyl, $C_1$-$C_6$-Alkoxy-$C_1$--$C_6$-alkyl, $C_1$-$C_4$-Alkanoyloxy-$C_1$-$C_6$-alkyl, Benzyloxy-$C_1$-$C_6$-alkyl oder gegebenenfalls durch Halogen substituiertes Phenyl-$C_1$-$C_6$-alkyl, $R^5$ $C_1$-$C_6$-Alkyl, $R^6$ Wasserstoff, $C_1$-$C_6$-Alkyl, $C_3$-$C_6$-Alkenyl oder Phenyl-$C_1$-$C_6$-alkyl, $R^7$ Wasserstoff oder $C_1$-$C_6$--Alkyl oder $R^6$ und $R^7$ zusammen eine $-(CH_2)_n$-Gruppe, worin n die Zahl 2 oder 3 bedeutet, und R Naphthyl, gegebenenfalls durch $C_1$-$C_6$-Alkyl, Halogen, Trifluormethyl oder Nitro monosubstituiertes oder durch Halogen bzw. Halogen und Nitro disubstituiertes Phenyl, Imidazolyl oder

Pyridyl bedeuten.

Eine bevorzugte Klasse von Verbindungen der Formel I sind solche, worin A die Gruppe $-CH(R^7)-CO-$ bedeutet. Weiters sind solche Verbindungen der Formel I bevorzugt, worin R Aryl, vorzugsweise durch $C_1$-$C_6$-Alkyl, Halogen, Trifluormethyl oder Nitro substituiertes Phenyl, bedeutet. Ganz besonders bevorzugt bedeutet R 3-Nitrophenyl, 2-Chlor-5-nitrophenyl oder 2,5-Dichlorphenyl. $R^1$ bedeutet vorzugsweise Methyl. Die bevorzugte Bedeutung von $R^2$ ist Wasserstoff. $R^3$ bedeutet vorzugsweise $C_1$-$C_4$-Alkyl, besonders bevorzugt Methyl. Weitere bevorzugte Verbindungen der Formel I sind solche, worin $R^4$ $C_1$-$C_6$-Alkyl, Cyan-$C_2$-$C_6$-alkyl, Halogen-$C_2$-$C_6$-alkyl, Hydroxy-$C_2$-$C_6$-alkyl, $\omega,\omega,\omega$-Trifluor-$C_1$-$C_6$-alkyl, $C_1$-$C_6$-Alkoxy-$C_1$-$C_6$-alkyl, $C_1$-$C_4$-Alkanoyloxy-$C_1$-$C_6$-alkyl, Benzyloxy-$C_1$-$C_6$-alkyl oder gegebenenfalls durch Halogen substituiertes Phenyl-$C_1$-$C_6$-alkyl, vorzugsweise $C_1$-$C_6$-Alkyl, $\omega,\omega,\omega$-Trifluor-$C_1$-$C_6$-alkyl, $C_1$-$C_6$-Alkoxy-$C_1$-$C_6$-alkyl oder Phenyl-$C_1$-$C_6$-alkyl, ganz besonders bevorzugt Isopropyl, 2,2,2-Trifluoräthyl, 2-Propoxyäthyl oder 1-Phenyläthyl, bedeutet. $R^5$ bedeutet vorzugsweise $C_1$-$C_6$-Alkyl, besonders bevorzugt Methyl, Aethyl oder Isopropyl. Die bevorzugte Bedeutung von $R^6$ und $R^7$ ist Wasserstoff.

Aus dem obigen folgt, dass diejenigen Verbindungen der Formel I ganz besonders bevorzugt sind, worin A die Gruppe $-CH(R^7)-CO-$, R 3-Nitrophenyl, 2-Chlor-5-nitrophenyl oder 2,5-Dichlorphenyl, $R^1$ und $R^3$ je Methyl, $R^2$, $R^6$ und $R^7$ je Wasserstoff, $R^4$ Isopropyl, 2,2,2-Trifluoräthyl, 2-Propoxyäthyl oder 1-Phenyläthyl und $R^5$ Methyl, Aethyl oder Isopropyl bedeuten.

Die bevorzugtesten Verbindungen der Formel I sind:

5-[(Aethylsulfamoyl)acetyl]-1,4-dihydro-2,6-dimethyl-4-(3-nitrophenyl)nicotinsäureisopropylester,

1,4-Dihydro-2,6-dimethyl-5-[(methylsulfamoyl)acetyl]-4-(3-nitrophenyl)nicotinsäureisopropylester,

1,4-Dihydro-2,6-dimethyl-5-[(methylsulfamoyl)acetyl]-4-(3-nitrophenyl)nicotinsäure-2,2,2-trifluoräthylester,

4-(2-Chlor-5-nitrophenyl)-1,4-dihydro-5-[(isopropylsulfamoyl)acetyl]-2,6-dimethylnicotinsäureisopropylester,

4-(2,5-Dichlorphenyl)-1,4-dihydro-5-[(isopropylsulfamoyl)acetyl]-2,6-dimethylnicotinsäureisopropylester,

1,4-Dihydro-5-[(isopropylsulfamoyl)acetyl]-2,6-dimethyl 4-(3-nitrophenyl)nicotinsäure-2-propoxyäthylester und

(S)-1,4-Dihydro-5-[(isopropylsulfamoyl)acetyl]-2,6-dimethyl-4-(3-nitrophenyl)nicotinsäure-(S)-1-phenyläthylester.

Die Verbindungen der obigen Formel I können erfindungsgemäss hergestellt werden, indem man

a) zur Herstellung von Verbindungen der Formel I, worin A die Gruppe $-CH(R^7)-CO-$ bedeutet, d.h. von Verbindungen der allgemeinen Formel

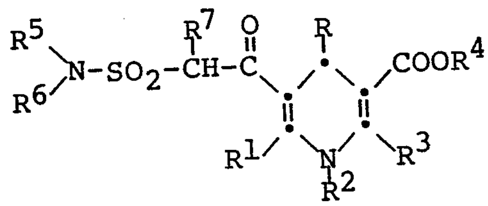

Ia

worin R, $R^1$, $R^2$, $R^3$, $R^4$, $R^5$, $R^6$ und $R^7$ die oben angegebene Bedeutung besitzen,

ein Enamin der allgemeinen Formel

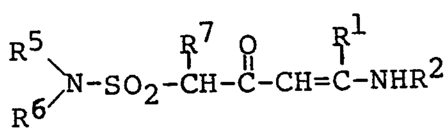

II

worin $R^1$, $R^2$, $R^5$, $R^6$ und $R^7$ die oben angegebene Bedeutung besitzen,

mit einer Ylidenverbindung der allgemeinen Formel

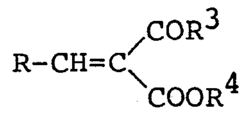

III

worin R, $R^3$ und $R^4$ die oben angegebene Bedeutung besitzen,

umsetzt, oder

b) zur Herstellung von Verbindungen der Formel I, worin A die Gruppe $-CH\overset{(E)}{=}CH-$ bedeutet, d.h. von Verbindungen der allgemeinen Formel

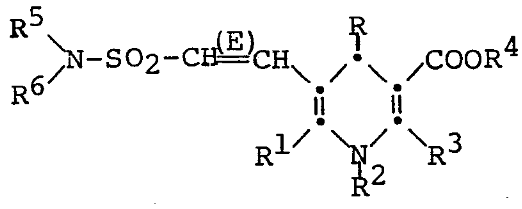

Ib

worin R, $R^1$, $R^2$, $R^3$, $R^4$, $R^5$, $R^6$ und $R^7$ die oben angegebene Bedeutung besitzen,

eine Verbindung der obigen Formel Ia, worin $R^7$ Wasserstoff bedeutet, mit Natriumborhydrid umsetzt, oder

c) erwünschtenfalls ein erhaltenes Isomerengemisch in die Isomeren auftrennt.

Die Umsetzung eines Enamins der Formel II mit einer Ylidenverbindung der Formel III gemäss Verfahrensvariante a) erfolgt nach an sich bekannten Methoden in Gegenwart eines inerten Lösungsmittels oder Lösungsmittelgemisches bei einer Temperatur zwischen etwa 20° und 150°C, vorzugsweise bei der Rückflusstemperatur des Lösungsmittels oder Lösungsmittelgemisches. Für diesen Zweck geeignete Lösungsmittel sind beispielsweise Alkohole, wie Methanol, Aethanol oder Isopropanol, Aether, wie Diäthyläther, Dioxan, Tetrahydrofuran, Glykolmonomethyläther oder Glykoldimethyläther, Eisessig, Dimethylformamid, Dimethylsulfoxid, Acetonitril oder Pyridin. Obwohl der Druck nicht kritisch ist und man die Umsetzung ohne weiteres bei erhöhtem Druck durchführen kann, arbeitet man aus Zweckmäsigkeitsgründen vorzugsweise bei Normaldruck. Die beiden Ausgangsstoffe werden vorzugsweise in äquimolaren Mengen eingesetzt.

Die Enamine der Formel II sind neu und ebenfalls Gegenstand vorliegender Erfindung. Die Enamine der Formel II, worin $R^6$ Wasserstoff bedeutet, d.h. die Verbindungen der allgemeinen Formel

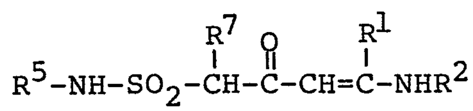

IIa

worin $R^1$, $R^2$, $R^5$ und $R^7$ die oben angegebene Bedeutung besitzen,

können aus 1,2-Thiazin-5(6H)-on-1,1-dioxiden der allgemeinen Formel

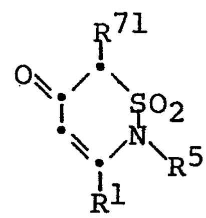

IV

worin $R^1$ und $R^5$ die oben angegebene Bedeutung besitzen und $R^{71}$ Wasserstoff, $C_1$-$C_6$-Alkyl oder gegebenenfalls durch $C_1$-$C_6$-Alkyl, $C_1$-$C_6$-Alkoxy oder Halogen substituiertes Phenyl-$C_1$-$C_6$-alkyl bedeutet,

durch Umsetzen mit einem Amin der allgemeinen Formel

$$R^2-NH_2 \qquad V$$

worin $R^2$ die oben angegebene Bedeutung besitzt,

hergestellt werden. Die Umsetzung erfolgt nach an sich

bekannten Methoden in Gegenwart eines inerten Lösungsmittels, wie einem Alkohol, beispielsweise Methanol, Aethanol oder Propanol, vorzugsweise Methanol, bei einer Temperatur zwischen etwa 0° und 40°C, vorzugsweise bei Raumtemperatur. Die 1,2-Thiazin-5(6H)-on-1,1-dioxide der Formel IV, worin $R^{71}$ Wasserstoff bedeutet, sind bekannt oder können in analoger Weise wie in der Literatur beschrieben hergestellt werden. Diejenigen, worin $R^{71}$ von Wasserstoff verschieden ist, können durch Alkylierung derjenigen, worin $R^{71}$ Wasserstoff bedeutet, d.h. von 1,2-Thiazin-5(6H)--on-1,1-dioxiden der allgemeinen Formel

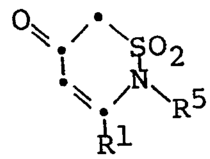

IVa

worin $R^1$ und $R^5$ die oben angegebene Bedeutung besitzen,

mit einer Verbindung der allgemeinen Formel

$$R^{72}-X \qquad VI$$

worin $R^{72}$ $C_1$-$C_6$-Alkyl oder gegebenenfalls durch $C_1$-$C_6$-Alkyl, $C_1$-$C_6$-Alkoxy oder Halogen substituiertes Phenyl-$C_1$-$C_6$-alkyl und X eine Abgangsgruppe bedeuten,

erhalten werden.

Die Alkylierung mit einer Verbindung der Formel VI erfolgt nach an sich bekannter Methoden, beispielsweise in Gegenwart einer Base, wie Natrium- oder Kaliumcarbonat oder Kalium-tert.-butylat, in einem Lösungsmittel, wie tert. Butanol, Benzol, Toluol, Diäthyläther, Dioxan, Tetrahydrofuran, Dimethylformamid, Dimethylsulfoxid und dergleichen, bei einer Temperatur zwischen etwa 0° und 50°C, vorzugsweise bei Raumtemperatur. Falls in der Verbindung der Formel VI $R^{71}$ $C_1$-$C_6$-Alkyl und X Halogen bedeuten, es

sich also bei der Verbindung der Formel VI um ein Alkylhalogenid handelt, kann die Alkylierung auch in einem Zweiphasen-System in Gegenwart einer Base und eines Phasentransfer-Katalysators durchgeführt werden, beispielsweise mit einem Alkyljodid in Methylenchlorid/wässrige Natronlauge in Gegenwart von Tetrabutylammonium-hydrogensulfat bei einer Temperatur zwischen etwa 10° und 20°C.

Die Enamine der Formel II, worin $R^6$ $C_1$-$C_6$-Alkyl, $C_3$-$C_6$-Alkenyl, $C_3$-$C_6$-Alkinyl, $C_3$-$C_6$-Cycloalkyl, $C_3$-$C_6$-Cycloalkyl-$C_1$-$C_6$-alkyl oder gegebenenfalls durch $C_1$-$C_6$-Alkyl, $C_1$-$C_6$-Alkoxy oder Halogen substituiertes Phenyl-$C_1$-$C_6$-alkyl bedeutet, können durch Umsetzen eines Enamins der obigen Formel IIa mit einer Verbindung der allgemeinen Formel

$$R^{61}\text{-}X \qquad\qquad VII$$

worin $R^{61}$ $C_1$-$C_6$-Alkyl, $C_3$-$C_6$-Alkenyl, $C_3$-$C_6$-Alkinyl, $C_3$-$C_6$-Cycloalkyl, $C_3$-$C_6$--Cycloalkyl-$C_1$-$C_6$-alkyl oder gegebenenfalls durch $C_1$-$C_6$-Alkyl, $C_1$-$C_6$-Alkoxy oder Halogen substituiertes Phenyl-$C_1$-$C_6$-alkyl bedeutet und X die oben angegebene Bedeutung besitzt,

nach an sich bekannten Methoden hergestellt werden, vorzugsweise durch Umsetzen mit einer Verbindung der Formel VII, worin X Halogen bedeutet, in Gegenwart einer Base, wie Kaliumcarbonat, in einem inerten Lösungsmittel, wie Dimethylformamid, bei Raumtemperatur.

Die Enamine der Formel II, worin $R^6$ und $R^7$ zusammen eine $-(CH_2)_n$-Gruppe bedeuten, können in ähnlicher Weise wie oben für die Umsetzung eines Enamins der Formel IIa mit einer Verbindung der Formel VII beschrieben aus den Enaminen der Formel IIa und einer Verbindung der allgemeinen Formel

$$X\text{-}(CH_2)_n\text{-}X \qquad VIII$$

worin X und n die oben angegebene Bedeutung besitzen, hergestellt werden.

Die Ylidenverbindungen der Formel III sind bereits bekannt oder können nach bekannten Methoden erhalten werden, beispielsweise durch Umsetzen eines ß-Ketocarbonsäureesters der allgemeinen Formel

$$R^3\text{-}\overset{O}{\overset{\|}{C}}\text{-}CH_2\text{-}COOR^4 \qquad IX$$

worin $R^3$ und $R^4$ die oben angegebene Bedeutung besitzen,

mit einem Aldehyd der allgemeinen Formel

$$RCHO \qquad X$$

worin R die oben angegebene Bedeutung besitzt.

Die erhaltene Ylidenverbindung der Formel III braucht vor deren Umsetzung mit einem Enamin der Formel II nicht isoliert zu werden; vielmehr kann man zur Herstellung von Verbindungen der Formel Ia die Verbindungen der Formeln II, IX und X, vorzugsweise in äquimolaren Mengen, unter den für die Umsetzung eines Enamins der Formel II mit einer Ylidenverbindung der Formel III beschriebenen Reaktionsbedingungen, miteinander umsetzen, wobei man direkt zur erwünschten Verbindung der Formel Ia gelangt.

Die Ausgangsstoffe der obigen Formeln IX und X sind bekannt oder können in analoger Weise zur Herstellung der bekannten Verbindungen erhalten werden.

Die Umsetzung einer Verbindung der Formel Ia, worin $R^7$ Wasserstoff bedeutet, mit Natriumborhydrid unter

gleichzeitiger Reduktion und Dehydratisierung gemäss Verfahrensvariante b) erfolgt nach an sich bekannten Methoden, zweckmässigerweise in einem organischen Lösungsmittel, wie einem Alkohol, beispielsweise Aethanol, Propanol, Isopropanol und dergleichen, oder einem Gemisch eines Alkohols mit Dimethylformamid oder 1,2-Dimethoxyäther, bei einer Temperatur zwischen etwa 0° und 50°C, vorzugsweise bei Raumtemperatur.

Die Verbindungen der allgemeinen Formel I enthalten mindestens ein asymmetrisches Zentrum (4-Stellung) und können deshalb als optische Antipoden oder als Racemate vorliegen. Verbindungen der Formel I, die mehr als ein asymmetrisches Zentrum enthalten, können in verschiedenen diastereoisomeren Formen vorliegen. Die vorliegende Erfindung umfasst sämtliche möglichen Stereoisomeren von Verbindungen der allgemeinen Formel I und alle möglichen diastereoisomeren Mischungen und Racemate, sowie die Auftrennung dieser diastereoisomeren Mischungen, die nach an sich bekannten Methoden durchgeführt werden kann.

Die Verbindungen der Formel I besitzen eine ausgeprägte Calcium-antagonistische Wirkung und können deshalb als Arzneimittel verwendet werden, insbesondere für die Bekämpfung bzw. Verhütung von Angina pectoris, Ischämie, Bluthochdruck und Migräne.

Die Calcium-antagonistische Wirkung sowie die blutdrucksenkenden Eigenschaften der erfindungsgemässen Verbindungen können in den nachstehend beschriebenen Tests gezeigt werden:

A. $^3$H-Nifedipin-Bindungsbestimmungen:

Die Bestimmung wird an Homogenaten oder an partiell-gereinigten Membranen vom Kaninchen- oder Meerschweinchen--Herz durchgeführt. Die Reaktionsmischung (0,3 ml) besteht aus 0,2-0,8 mg Membranprotein, 1 nM $^3$H-Nifedipin (oder

0,25 nM $^3$H-Nitrendipin) und verschiedenen Konzentrationen der Prüfsubstanzen. Die Inkubation dauert 30 Minuten bei 25°C oder 37°C und wird durch Verdünnung mit dem Inkubationspuffer gestoppt; anschliessend erfolgt eine Filtration. Die filtergebundene Radioaktivität wird mit einem Szintillationszähler gemessen. Spezifische Bindung (d.h. rezeptorgebundene) wird als die Differenz zwischen total- und unspezifischgebundener Radioaktivität definiert. Die unspezifische Bindung wird in Gegenwart von einem Ueberschuss nichtradioaktivem Nifedipin (1µM) bestimmt.

Die Wirksamkeit (Potenz) einer Verbindung in diesem Test wird durch die $IC_{50}$- und % Maximale Hemmungs-Werte (% max. Inhibition) definiert. $IC_{50}$ ist die Substanzkonzentration (in Mol/l), die eine halb-maximale Inhibition der spezifischen $^3$H-Nifedipin (bzw. $^3$H-Nitrendipin)--Bindung verursacht. Die maximale Hemmung der spezifischen Bindung wird durch den % Maximale-Hemmungs-Wert angegeben; für die Referenzverbindung Nifedipin ist dieser Wert als 100% festgesetzt. Beide Parameter werden aus einer Konzentration-Bindung-Kurve extrapoliert.

B. <u>Hunde-Koronararterienstreifen:</u>
In diesem Versuch werden spirale Streifen (2-2,5 mm breit und 10 mm lang) von Hunde-Koronararterien geschnitten und in einer Organkammer unter 1,5 g Vorspannung aufgehängt. Diese Streifen werden ca. 1 bis 2 Stunden lang in Krebs--Henseleit-Pufferlösung vorinkubiert, die bei 37°C mit Oxycarbon (Gemisch von 95% Sauerstoff und 5% Kohlendioxyd) begast wird. Anschliessend wird die relaxierende Wirkung einer dieser Substanzen auf einer KCl (84,7 mM)-Kontraktur geprüft, durch Zugabe steigender Konzentrationen der Prüfsubstanz in die Organkammer. Die Calcium-Kanal-blockierende Wirkung der Prüfsubstanzen kann deshalb festgestellt werden, weil die KCl-Kontraktur ausschliesslich mittels Calcium-Einstrom durch den spannungsabhängigen Calcium-Kanal erfolgt.

Die Wirksamkeit einer Prüfsubstanz in diesem Test wird durch den $IC_{50}$-Wert angegeben. Dieser Wert wird als die Substanzkonzentration (in Mol/l) definiert, die eine halb--maximale Relaxation einer KCl-Kontraktur verursacht. Dieser Wert wird auch aus der resultierenden Konzentration--Wirkung-Kurve extrapoliert.

C. <u>Hämodynamische Parameter am narkotisierten Hund:</u>
Die 4 wichtigsten Messparameter (mit resp. Messeinheiten) der hämodynamischen Versuche sind: (1) CBF: Coronary Blood Flow (in ml/min) - die Blutflussgeschwindigkeit durch die Koronararterien; (2) HR: Heart Rate (in Schläge/min) - die Herzfrequenz; (3) BP: Blood Pressure (in mm Hg) - der Blutdruck; und (4) dp/dt: Rate of increase in left ventricular pressure (in mm Hg/sec) - die Anstiegsgeschwindigkeit des linksventrikulären Druckes, als Mass für die Kontraktilitätskraft des Herzens. Die Werte werden als % maximale Aenderung vom Ausgangswert ($\Delta$%) und Zeitdauer dieser Aenderung (t) pro verabreichter Dosis angegeben.

Dadurch bekommt man nicht nur ein Gesamtbild der Substanzwirkung, sondern auch eine Abschätzung über die potentielle Selektivität für einen bestimmten Teil des Kreislaufsystems im ganzen Organismus. Nach Verabreichung eines Anästhetikums, wird der Hund intubiert und künstlich beatmet. Blut pH, $pCO_2$, $pO_2$ und Hämoglobin werden mit einem Blut-Gas-Analysator stündlich gemessen. Der Blutdruck (systolisch und diastolisch) wird mit einer Sonde in der Aorta abdominalis gemessen. Die Herzfrequenz wird mittels eines Tachometers erfasst, der vom Druckpuls ausgelöst wird. Für die anderen Messungen muss das Herz zuerst freigelegt werden, um eine Sonde in den linken Ventrikel (Herzkammer) für die Druckmessungen (dp/dt) einsetzen zu können. Der Coronarblutfluss wird mit einer Fluss-Sonde an der linken Coronararterie (descendens) gemessen.

Die in diesen Tests erhaltenen Resultate sind in der folgenden Tabelle zusammengefasst:

Tabelle

| Verbindung | A | | B | C | | | | |
|---|---|---|---|---|---|---|---|---|
| | $IC_{50}$ [M] | % max. Inhib. | $IC_{50}$ [M] | CBF ml/min. t | HR Schläge/min. t | BP mmHg t | dp/dt mmHg/sec. t | Dosis mg/kg p.o. |
| A | $3,0 \cdot 10^{-8}$ | 100 | $6,8 \cdot 10^{-9}$ | 98(90') | -11(60') | -13(> 60') | 37( 90') | 0,03 |
| B | $8,4 \cdot 10^{-8}$ | 94 | $1,6 \cdot 10^{-8}$ | 120(> 90') | 0 - | - 8(> 90') | 10(> 90') | 0,03 |
| C | $4,2 \cdot 10^{-8}$ | 100 | $9,5 \cdot 10^{-9}$ | 88( 180') | +2(120') | -16(>180') | 33( 180') | 0,1 |
| D | $1,5 \cdot 10^{-7}$ | 100 | $8,2 \cdot 10^{-10}$ | 68(>120') | -5( 60') | - 9(> 60') | 75( 90') | 0,03 |
| E | $2,9 \cdot 10^{-8}$ | 100 | $6,6 \cdot 10^{-9}$ | 104( 70') | -14(120') | -16(>120') | 65( 90') | 0,03 |
| F | $2,7 \cdot 10^{-9}$ | 99 | $6,0 \cdot 10^{-10}$ | 56( 120') | -5( 16') | -11(> 60') | 34( 60') | 0,03 |
| G | $4 \cdot 10^{-9}$ | 100 | $7,4 \cdot 10^{-9}$ | 57(>120') | -13(>120') | -36(>120') | 29(>120') | 0,03 |

A = 5-[(Aethylsulfamoyl)acetyl]-1,4-dihydro-2,6-dimethyl-4-(3-nitrophenyl)nicotinsäureisopropylester

B = 1,4-Dihydro-2,6-dimethyl-5-[(methylsulfamoyl)acetyl]-4-(3-nitrophenyl)nicotinsäureisopropylester

C = 1,4-Dihydro-2,6-dimethyl-5-[(methylsulfamoyl)acetyl]-4-(3-nitrophenyl)nicotinsäure-2,2,2-trifluoräthylester

D = 4-(2-Chlor-5-nitrophenyl)-1,4-dihydro-5-[(isopropylsulfamoyl)acetyl]-2,6-dimethylnicotinsäureisopropylester

E = 4-(2,5-Dichlorphenyl)-1,4-dihydro-5-[(isopropylsulfamoyl)acetyl]-2,6-dimethylnicotinsäureisopropylester

F = 1,4-Dihydro-5-[(isopropylsulfamoyl)acetyl]-2,6-dimethyl-4-(3-nitrophenyl)nicotinsäure-2-propoxyäthylester

G = (S)-1,4-Dihydro-5-[(isopropylsulfamoyl)acetyl]-2,6-dimethyl-4-(3-nitrophenyl)nicotinsäure-(S)-1-phenyläthylester

Die Verbindungen der Formel I können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragées und Hartgelatinekapseln können die Verbindungen der Formel I mit pharmazeutisch inerten, anorganischen oder organischen Excipientien verarbeitet werden. Als solche Excipientien kann man z.B. für Tabletten, Dragées und Hartgelatinekapseln, Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie

können auch noch andere therapeutisch wertvolle Stoffe enthalten.

Erfindungsgemäss kann man Verbindungen der allgemeinen Formel I bei der Bekämpfung bzw. Verhütung von Angina pectoris, Ischämie, Bluthochdruck und Migräne verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 10 bis 100 mg einer Verbindung der allgemeinen Formel I angemessen sein, wobei aber die soeben angegebene obere Grenze auch überschritten werden kann, wenn sich dies als angezeigt erweisen sollte.

Die folgenden Beispiele sollen die vorliegende Erfindung erläutern, sie jedoch in keiner Weise einschränken. Alle Temperaturen sind in Celsius Graden angegeben.

## Beispiel 1

Eine Lösung von 4,88 g (0,02 Mol) 3-Nitrobenzylidenacetessigsäuremethylester in einer Mischung von 20 ml Isopropanol und 10 ml Dimethylformamid wird unter Argon mit 3,84 g (0,02 Mol) 4-Amino-N-methyl-2-oxo-3-pentensulfonamid versetzt und hierauf 8 Stunden zum Rückfluss erhitzt. Die Lösung wird dann unter vermindertem Druck zur Trockene eingeengt, und das verbleibende gelbe Oel an 500 g Kieselgel mit Essigester als Eluierungsmittel chromatographiert. Die einheitlichen Fraktionen werden vereinigt und eingedampft. Nach dem Anreiben des Rückstandes mit Aether erhält man 3,8 g (45%) 1,4-Dihydro-2,6-dimethyl-5-[(methylsulfamoyl)-acetyl]-4-(3-nitrophenyl)nicotinsäuremethylester, Smp. 173-176°, als gelbes Kristallpulver. Umkristallisation aus Aethanol erhöht den Schmelzpunkt nicht.

Das als Ausgangsmaterial verwendete 4-Amino-N-methyl--2-oxo-3-pentensulfonamid kann wie folgt hergestellt werden:

In eine Suspension von 10 g 2,3-Dimethyl-1,2-thiazin--5(6H)-on-1,1-dioxid in 100 ml Methanol wird während 3 Stunden bei 20-25° ein mässig rascher Strom von trockenem Ammoniak eingeleitet, wobei nach ca. 10-15 Minuten eine klare hellgelbe Lösung entsteht. Hierauf wird über Nacht bei Raumtemperatur weiter gerührt. Das auskristallisierte Produkt wird abfiltriert und mit wenig Methanol gewaschen, wobei man 9,8 g 4-Amino-N-methyl-2-oxo-3-pentensulfonamid in Form von farblosen Kristallen erhält, Smp. 158-162°. Aus dem Filtrat kann man durch Einengen auf ca. 50 ml zusätzliche 2,2 g Produkt erhalten, Smp. 158-162°, Gesamtausbeute: 12 g (94%).

## Beispiel 2

In analoger Weise wie in Beispiel 1 beschrieben können die folgenden Verbindungen hergestellt werden:

- 1,4-Dihydro-2,6-dimethyl-5-[(methylsulfamoyl)-acetyl]-4-(3-nitrophenyl)nicotinsäure-2-chloräthylester, Smp. 101-105°, aus 3-Nitrobenzylidenacetessigsäure-2--chloräthylester und 4-Amino-N-methyl-2-oxo-3-pentensulfonamid;
- 1,4-Dihydro-2,6-dimethyl-5-[(methylsulfamoyl)-acetyl]-4-(3-nitrophenyl)nicotinsäure-2-propoxyäthylester, Smp. 106-110°, aus 3-Nitrobenzylidenacetessigsäure-2-propoxyäthylester und 4-Amino-N-methyl-2-oxo--3-pentensulfonamid;
- 1,4-Dihydro-2,6-dimethyl-5-[(methylsulfamoyl)-acetyl]-4-(3-nitrophenyl)nicotinsäure-3-chlorpropylester, Smp. 96-101° (kristallisiert mit 0,5 Mol Aethanol), aus 3-Nitrobenzylidenacetessigsäure-3-chlorpropylester und 4-Amino-N-methyl-2-oxo-3-pentensulfonamid;
- 1,4-Dihydro-2,6-dimethyl-5-[(methylsulfamoyl)acetyl]-4--(3-nitrophenyl)nicotinsäureisopropylester, Smp. 177-180°

aus 3-Nitrobenzylidenacetessigsäureisopropylester und 4-Amino-N-methyl-2-oxo-3-pentensulfonamid;

- 1,4-Dihydro-2,6-dimethyl-5-[(methylsulfamoyl)acetyl]--4-(3-nitrophenyl)nicotinsäure-2,2,2-trifluoräthylester, Smp. 135-140° (kristallisiert mit 0,5 Mol Aethanol), aus 3-Nitrobenzylidenacetessigsäure-2,2,2-trifluoräthylester und 4-Amino-N-methyl-2-oxo-3-pentensulfonamid und
- 1,4-Dihydro-2,6-dimethyl-5-[(methylsulfamoyl)acetyl]--4-(2-nitrophenyl)nicotinsäureisopropylester, Smp. 156-158° (kristallisiert mit 0,5 Mol Aethanol), aus 2-Nitrobenzylidenacetessigsäureisopropylester und 4-Amino-N-methyl-2-oxo-3-pentensulfonamid.

## Beispiel 3

Eine Lösung von 5,54 g (0,02 Mol) 3-Nitrobenzylidenacetessigsäureisopropylester in 20 ml Isopropanol und 10 ml Dimethylformamid wird unter Argon mit 4,12 g (0,02 Mol) N-Aethyl-4-amino-2-oxo-3-pentensulfonamid versetzt und 8 Stunden zum Rückfluss erhitzt. Das Gemisch wird hierauf unter vermindertem Druck zur Trockene eingeengt, und der ölige Rückstand an 500 g Kieselgel mit Methylenchlorid/- Essigester (4:1) als Eluierungsmittel chromatographiert. Die homogenen Fraktionen werden vereinigt und eingedampft. Das verbleibende Oel kristallisiert beim Anreiben mit Aether. Umkristallisation aus Aethanol/Aether liefert 5,0 g (54%) 5-[(Aethylsulfamoyl)-acetyl]-1,4-dihydro-2,6-dimethyl-4-(3-nitrophenyl)nicotinsäureisopropylester in Form von gelben Kristallen, Smp. 140-142°.

Das als Ausgangsmaterial verwendete N-Aethyl-4-amino--2-oxo-3-pentensulfonamid kann wie folgt hergestellt werden:

Eine auf -75° gekühlte Lösung von 24,5 g (0,1 Mol) 1-Methyl-1,3-bis(trimethylsiloxy)-1,3-butadien und 10,1 g (0,1 Mol) Triäthylamin in 100 ml trockenem Tetrahydrofuran wird unter Argon bei -80° bis -70° tropfenweise mit einer

Lösung von 14,4 g (0,1 Mol) N-Aethyl-sulfamoylchlorid in 50 ml trockenem Tetrahydrofuran versetzt und anschliessend 2 Stunden bei derselben Temperatur gerührt. Man lässt dann die Temperatur auf Raumtemperatur steigen, rührt 1 Stunde, säuert bei 15-20° mit 110 ml 2N Salzsäure an und rührt 15 Minuten weiter. Das Gemisch wird mit Aether extrahiert, und die über Natriumsulfat getrockneten Extrakte zur Trockene eingedampft. Man löst das verbleibende Oel in 250 ml Toluol, gibt 0,4 g Methansulfonsäure zu und erhitzt 15 Stunden zum Rückfluss mit einem Wasserabscheider. Die erhaltene Lösung wird abgekühlt, unter vermindertem Druck zur Trockene eingeengt, und das verbleibende Oel an 280 g Kieselgel mit Methylenchlorid/Essigester (9:1) als Eluierungsmittel chromatographiert. Die einheitlichen Fraktionen werden vereinigt und eingedampft. Nach dem Anreiben des Rückstandes mit Aether erhält man 13,8 g (73%) 2-Aethyl-3-methyl-1,2-thiazin-5(6H)-on-1,1-dioxid in Form von fast farblosen Kristallen, Smp. 90-92°. Umkristallisation aus Aethanol erhöht den Schmelzpunkt nicht.

Gemäss der in Beispiel 1 beschriebenen Arbeitsweise wird eine Suspension von 13 g 2-Aethyl-3-methyl-1,2-thiazin-5(6H)--on-1,1-dioxid in 130 ml Methanol mit Ammoniak umgesetzt. Die erhaltene dunkelgelbe Lösung wird unter vermindertem Druck zur Trockene eingeengt, und der kristalline Rückstand aus Aethanol umkristallisiert. Man erhält 12,2 g (86%) N-Aethyl-4-amino-2--oxo-3-pentensulfonamid in Form von farblosen Kristallen, Smp. 112-114°.

## Beispiel 4

Eine Lösung von 5,54 g (0,02 Mol) 3-Nitrobenzylidenacetessigsäureisopropylester in einer Mischung von 50 ml Isopropanol und 24 ml Dimethylformamid wird unter Argon mit 4,40 g (0,02 Mol) 4-Amino-N-isopropyl-2-oxo-3-pentensulfonamid versetzt und hierauf 16 Stunden zum Rückfluss erhitzt. Nach dem Einengen unter vermindertem Druck wird der ölige Rückstand an 300 g Kieselgel mit Methylenchlorid/Essigester (4:1) als

Eluierungsmittel chromatographiert. Die homogenen Fraktionen liefern ein Oel, welches durch Anreiben mit Aether kristallisiert. Man erhält 10,8 g (56%) 1,4-Dihydro-5-[(isopropylsulfamoyl)acetyl]-2,6-dimethyl-4-(3-nitrophenyl)nicotinsäureisopropylester in Form von gelben Kristallen, Smp. 119-121°. Umkristallisation aus Methylenchlorid/Aether erhöht den Schmelzpunkt nicht.

Das als Ausgangsmaterial verwendete 4-Amino-N-isopropyl-2-oxo-3-pentensulfonamid kann wie folgt hergestellt werden:

Gemäss der in Beispiel 1 beschriebenen Arbeitsweise wird eine Suspension von 10 g 2-Isopropyl-3-methyl-1,2-thiazin-5(6H)-on-1,1-dioxid in 100 ml Methanol mit Ammoniak umgesetzt. Die erhaltene Lösung wird unter vermindertem Druck zur Trockene eingeengt, und der feste Rückstand aus Isopropanol umkristallisiert. Man erhält 10,1 g (93%) 4-Amino-N-isopropyl-2-oxo-3-pentensulfonamid in Form von farblosen Kristallen, Smp. 119-121°.

## Beispiel 5

In analoger Weise wie in Beispiel 4 beschrieben können die folgenden Verbindungen hergestellt werden:

- 1,4-Dihydro-5-[(isopropylsulfamoyl)acetyl]-2,6-dimethyl-4-(3-nitrophenyl)nicotinsäuremethylester, Smp. 208-212°, aus 3-Nitrobenzylidenacetessigsäuremethylester und 4-Amino-N-isopropyl-2-oxo-3-pentensulfonamid;
- 1,4-Dihydro-5-[(isopropylsulfamoyl)acetyl]-2,6-dimethyl-4-(3-trifluormethylphenyl)nicotinsäuremethylester, Smp. 184-187°, aus 3-Trifluormethylbenzylidenacetessigsäuremethylester und 4-Amino-N-isopropyl-2-oxo-3-pentensulfonamid;
- 1,4-Dihydro-5-[(isopropylsulfamoyl)acetyl]-2,6-dimethyl-4-(3-nitrophenyl)nicotinsäure-2-chloräthylester,

Smp. 121-125°, aus 3-Nitrobenzylidenacetessigsäure-2--chloräthylester und 4-Amino-N-isopropyl-2-oxo-3-pentensulfonamid;

- 1,4-Dihydro-5-[(isopropylsulfamoyl)acetyl]-2,6--dimethyl-4-(3-nitrophenyl)nicotinsäureisobutylester, Smp. 75-79°, aus 3-Nitrobenzylidenacetessigsäureisobutylester und 4-Amino-N-isopropyl-2-oxo-3-pentensulfonamid;
- 4-(2,3-Dichlorphenyl)-1,4-dihydro-5-[(isopropylsulfamoyl)acetyl]-2,6-dimethylnicotinsäureisopropylester, Smp. 152-154°, aus 2,3-Dichlorbenzylidenacetessigsäureisopropylester und 4-Amino-N-isopropyl-2-oxo-3--pentensulfonamid;
- 1,4-Dihydro-5-[(isopropylsulfamoyl)acetyl]-2,6--dimethyl-4-(3-nitrophenyl)nicotinsäureäthylester, Smp. 124-126°, aus 3-Nitrobenzylidenacetessigsäureäthylester und 4-Amino-N-isopropyl-2-oxo-3-pentensulfonamid;
- 1,4-Dihydro-5-[(isopropylsulfamoyl)acetyl]-2,6--dimethyl-4-(3-nitrophenyl)nicotinsäurepropylester, Smp. 76-80°, aus 3-Nitrobenzylidenacetessigsäurepropylester und 4-Amino-N-isopropyl-2-oxo-3-pentensulfonamid;
- 1,4-Dihydro-5-[(isopropylsulfamoyl)acetyl]-2,6--dimethyl-4-(3-nitrophenyl)nicotinsäure-2-(benzyloxy)-äthylester, Smp. 144-147°, aus 3-Nitrobenzylidenacetessigsäure-2-(benzyloxy)äthylester und 4-Amino-N-isopropyl-2-oxo-3-pentensulfonamid;
- 1,4-Dihydro-5-[(isopropylsulfamoyl)acetyl]-2,6--dimethyl-4-(3-nitrophenyl)nicotinsäure-2,2,2-trifluoräthylester, Smp. 138-141°, aus 3-Nitrobenzylidenacetessigsäure-2,2,2-trifluoräthylester und 4-Amino-N-isopropyl-2-oxo-3-pentensulfonamid;
- 1,4-Dihydro-5-[(isopropylsulfamoyl)acetyl]-4-(imidazol--2-yl)-2,6-dimethylnicotinsäureisopropylester, Smp. 227-229°, aus 2-Imidazolyl-methylenacetessigsäureisopropylester und 4-Amino-N-isopropyl-2-oxo-3-pentensulfonamid;
- 4-(2-Chlor-5-nitrophenyl)-1,4-dihydro-5-[(isopropylsul-

famoyl)acetyl]-2,6-dimethylnicotinsäureisopropylester, Smp. 195-197°, aus 2-Chlor-5-nitrobenzylidenacetessigsäureisopropylester und 4-Amino-N-isopropyl-2-oxo-3--pentensulfonamid;

- 1,4-Dihydro-5-[(isopropylsulfamoyl)acetyl]-2,6--dimethyl-4-(2-nitrophenyl)nicotinsäureisopropylester, Smp. 175-177° (kristallisiert mit 0,5 Mol Aethanol), aus 2-Nitrobenzylidenacetessigsäureisopropylester und 4-Amino-N-isopropyl-2-oxo-3-pentensulfonamid;
- 1,4-Dihydro-5-[(isopropylsulfamoyl)acetyl]-2,6--dimethyl-4-(1-naphthyl)nicotinsäureisopropylester, Smp. 187-189°, aus 1-Naphthyl-methylenacetessigsäureisopropylester und 4-Amino-N-isopropyl-2-oxo-3-pentensulfonamid;
- 4-(2,5-Dichlorphenyl)-1,4-dihydro-5-[(isopropylsulfamoyl)acetyl]-2,6-dimethylnicotinsäureisopropylester, Smp. 158-160°, aus 2,5-Dichlorbenzylidenacetessigsäureisopropylester und 4-Amino-N-isopropyl-2-oxo-3-pentensulfonamid;
- 1,4-Dihydro-5-[(isopropylsulfamoyl)acetyl]-2,6--dimethyl-4-(3-pyridyl)nicotinsäureisopropylester, Smp. 179-181°, aus 3-Pyridyl-methylenacetessigsäureisopropylester und 4-Amino-N-isopropyl-2-oxo-3-pentensulfonamid;
- 1,4-Dihydro-5-[(isopropylsulfamoyl)acetyl]-2,6--dimethyl-4-(2-tolyl)nicotinsäureisopropylester, Smp. 163-166°, aus 2-Methylbenzylidenacetessigsäureisopropylester und 4-Amino-N-isopropyl-2-oxo-3-pentensulfonamid;
- 1,4-Dihydro-5-[(isopropylsulfamoyl)acetyl]-2,6--dimethyl-4-(3-nitrophenyl)nicotinsäure-2-propoxyäthylester Smp. 103-106°, aus 3-Nitrobenzylidenacetessigsäure-2--propoxyäthylester und 4-Amino-N-isopropyl-2-oxo-3-pentensulfonamid;
- 1,4-Dihydro-5-[(isopropylsulfamoyl)acetyl]-2,6--dimethyl-4-(3-nitrophenyl)nicotinsäure-2-methoxyäthylester, Smp. 133-136°, aus 3-Nitrobenzylidenacetessi-

säure-2-methoxyäthylester und 4-Amino-N-isopropyl-2--oxo-3-pentensulfonamid und

- 4-(2-Chlor-5-nitrophenyl)-1,4-dihydro-5-[(isopropylsulfamoyl)acetyl]-2,6-dimethylnicotinsäure-2-cyanäthylester, Smp. 196-198°, aus 2-Chlor-5-nitrobenzylidenacetessigsäure-2-cyanäthylester und 4-Amino-N-isopropyl-2-oxo-3-pentensulfonamid.

## Beispiel 6

Nach 6-stündigem Kochen von 2,5 g (0,01 Mol) 3-Nitrobenzylidenacetessigsäuremethylester und 1,92 g (0,01 Mol) N-Aethyl-4-amino-2-oxo-3-butensulfonamid in einer Mischung von 10 ml Isopropanol und 5 ml Dimethylformamid werden 2,2 g (52%) 5-[(Aethylsulfamoyl)acetyl]-1,4-dihydro-2-methyl-4-(3-nitrophenyl)nicotinsäuremethylester vom Schmelzpunkt 185-187° (gelbes Kristallpulver aus Acetonitril) erhalten.

Zur Herstellung des als Ausgangsmaterial verwendeten N-Aethyl-4-amino-2-oxo-3-butensulfonamides werden gemäss der in Beispiel 1 beschriebenen Arbeitsweise 5 g 2-Aethyl--1,2-thiazin-5(6H)-on-1,1-dioxid in 50 ml Methanol mit Ammoniak umgesetzt. Man erhält 4,8 g (88%) farblose Kristalle, Smp. 96-98° (aus Aether).

## Beispiel 7

Nach 6-stündigem Kochen von 2,5 g (0,01 Mol) 3-Nitrobenzylidenacetessigsäuremethylester und 2,06 g (0,01 Mol) 4-Amino-N,N-dimethyl-2-oxo-3-pentensulfonamid in einer Mischung von 10 ml Isopropanol und 5 ml Dimethylformamid werden 2,3 g (52%) 1,4-Dihydro-2,6-dimethyl-5-[(dimethylsulfamoyl)acetyl]-4-(3-nitrophenyl)nicotinsäuremethylester vom Schmelzpunkt 154-157° (gelbes Kristallpulver aus Aethanol) erhalten.

Das als Ausgangsmaterial verwendete 4-Amino-N,N--dimethyl-2-oxo-3-pentensulfonamid kann wie folgt hergestellt werden:

Eine Lösung von 3,8 g (0,02 Mol) 4-Amino-N-methyl-2--oxo-3-pentensulfonamid und 1,9 ml (0,03 Mol) Methyljodid in 50 ml Dimethylformamid wird mit 6 g feingeriebenem trockenem Kaliumcarbonat versetzt und hierauf unter Argon während 6 Stunden bei Raumtemperatur intensiv gerührt. Die organischen Salze werden dann abfiltriert, mit Methylenchlorid nachgewaschen, und das Filtrat unter vermindertem Druck zur Trockene eingeengt. Der Rückstand wird zwischen Wasser und Methylenchlorid verteilt, die organische Phase mit einer gesättigten wässrigen Lösung von Natriumchlorid gewaschen, über Natriumsulfat getrocknet und zur Trockene eingedampft. Das feste Rohprodukt wird aus Isopropanol umkristallisiert, wobei man 2,5 g (61%) 4-Amino-N,N-dimethyl-2-oxo-3-pentensulfonamid in Form von farblosen Kristallen erhält, Smp. 137-140°.

## Beispiel 8

Nach 8-stündigem Kochen von 2,5 g (0,01 Mol) 3-Nitrobenzylidenacetessigsäuremethylester und 2,82 g (0,01 Mol) 4-Amino-N-benzyl-N-methyl-2-oxo-3-pentensulfonamid in einer Mischung von 16 ml Isopropanol und 8 ml Dimethylformamid werden 2,7 g (53%) 5-[(Benzylmethylsulfamoyl)acetyl]-1,4--dihydro-2,6-dimethyl-4-(3-nitrophenyl)nicotinsäuremethylester vom Schmelzpunkt 156-159° (gelbes Kristallpulver aus Acetonitril) erhalten.

Das als Ausgangsmaterial verwendete 4-Amino-N-benzyl--N-methyl-2-oxo-3-pentensulfonamid kann wie folgt hergestellt werden:

Gemäss der in Beispiel 7 beschriebenen Arbeitsweise wird eine Lösung von 3,8 g (0,02 Mol) 4-Amino-N-methyl-2--

oxo-3-pentensulfonamid und 3,6 ml (0,03 Mol) Benzylbromid in 50 ml Dimthylformamid mit 6 g Kaliumcarbonat versetzt und 9 Stunden bei Raumtemperatur intensiv gerührt. Das Rohprodukt wird zuerst an 300 g Kieselgel mit Essigester als Eluierungsmittel chromatographiert und dann aus Isopropanol umkristallisiert. Man erhält 3,4 g (61%) 4-Amino-N-benzyl--N-methyl-2-oxo-3-pentensulfonamid in Form von farblosen Kristallen, Smp. 115-118°.

## Beispiel 9

Nach 8-stündigem Kochen von 2,77 g (0,01 Mol) 3-Nitrobenzylidenacetessigsäureisopropylester und 2,32 g (0,01 Mol) N-Allyl-N-methyl-4-amino-2-oxo-3-pentensulfonamid in einer Mischung von 25 ml Isopropanol und 12 ml Dimethylformamid werden 1,9 g (39%) 5-[(Allylmethylsulfamoyl)acetyl]-1,4-dihydro--2,6-dimethyl-4-(3-nitrophenyl)nicotinsäureisopropylester vom Schmelzpunkt 137-140° (gelbes Kristallpulver aus Aethanol) erhalten.

Das als Ausgangsmaterial verwendete N-Allyl-N-methyl--4-amino-2-oxo-3-pentensulfonamid kann wie folgt hergestellt werden:

Gemäss der in Beispiel 7 beschriebenen Arbeitsweise wird eine Lösung von 3,8 g (0,02 Mol) 4-Amino-N-methyl-2--oxo-3-pentensulfonamid und 2,5 ml (0,03 Mol) Allylbromid in 50 ml Dimethylformamid mit 6 g Kaliumcarbonat versetzt und 7 Stunden bei Raumtemperatur intensiv gerührt. Das Rohprodukt wird zuerst an 300 g Kieselgel mit Methylenchlorid/-Essigester (4:1) als Eluierungsmittel chromatographiert und dann aus Methylenchlorid/Aether umkristallisiert. Man erhält 3,3 g (71%) N-Allyl-N-methyl-4-amino-2-oxo-3-pentensulfonamid in Form von farblosen Kristallen, Smp. 86-89°.

## Beispiel 10

Nach 8-stündigem Kochen von 2,98 g (0,01 Mol) 3-Nitrobenzylidenacetessigsäure-2-chloräthylester und 2,18 g (0,01 Mol) 5-(3-Aminocrotonyl)-2-methylthiazolidin-1,1-dioxid in einer Mischung von 15 ml Isopropanol und 5 ml Dimethylformamid werden 2,85 g (57%) 1,4-Dihydro-2,6-dimethyl-5-[(2'-methyl-5'-thiazolidinyl)carbonyl]-4-(3-nitrophenyl)nicotinsäure-2-chloräthylester-1',1'-dioxid als Diastereomerengemisch vom Schmelzpunkt 239-242° (gelbes Kristallpulver aus Acetonitril) erhalten.

Das als Ausgangsmaterial verwendete 5-(3-Aminocrotonyl)-2-methylthiazolidin-1,1-dioxid kann wie folgt hergestellt werden:

Gemäss der in Beispiel 7 beschriebenen Arbeitsweise wird eine Lösung von 3,8 g (0,02 Mol) 4-Amino-N-methyl-2-oxo-3-pentensulfonamid und 4 ml (0,046 Mol) Aethylenbromid in 50 ml Dimethylformamid mit 12 g Kaliumcarbonat versetzt und 6 Stunden im Oelbad bei 80° intensiv gerührt. Das Rohprodukt wird zuerst an 300 g Kieselgel mit Methylenchlorid/Essigester (1:1) als Eluierungsmittel chromatographiert und dann aus Aethanol umkristallisiert. Man erhält 1,9 g (43%) 5-(3-Aminocrotonyl)-2-methylthiazolidin-1,1-dioxid in Form von farblosen Kristallen, Smp. 113-115°.

## Beispiel 11

Nach 8-stündigem Kochen von 3,2 g (0,01 Mol) 3-Nitrobenzylidenacetessigsäure-2-propoxyäthylester und 2,05 g (0,01 Mol) 4-Amino-N,1-dimethyl-2-oxo-3-pentensulfonamid in einer Mischung von 10 ml Isopropanol und 5 ml Dimethylformamid werden 1,7 g (34%) 1,4-Dihydro-2,6-dimethyl-5-[2-(methylsulfamoyl)propionyl]-4-(3-nitrophenyl)nicotinsäure-2-propoxyäthylester erhalten, Smp. 120-123° (gelbes Kristallpulver aus Aethanol, nur eines der beiden möglichen

Racemate).

Das als Ausgangsmaterial verwendete 4-Amino-N,1--dimethyl-2-oxo-4-pentensulfonamid kann wie folgt hergestellt werden:

Zu einer Lösung von 3,5 g (0,02 Mol) 2,3-Dimethyl-1,2--thiazin-5(6H)-on-1,1-dioxid, 6,8 g (0,02 Mol) Tetrabutylammonium-hydrogensulfat und 1,9 ml (0,03 Mol) Methyljodid in 40 ml Methylenchlorid wird unter kräftigem Rühren bei 5-12° eine Lösung von 1,6 g (0,04 Mol) Natriumhydroxid in 20 ml Wasser getropft. Das Gemisch wird danach 15 Minuten bei Raumtemperatur kräftig weiter gerührt. Die organische Phase wird abgetrennt, die wässrige Phase zweimal mit 15 ml Methylenchlorid extrahiert, und die vereinigten organischen Phasen mit einer gesättigten wässrigen Lösung von Natriumchlorid gewaschen, über Natriumsulfat getrocknet und zur Trockene eingeengt. Der ölige Rückstand wird an 300 g Kieselgel mit Chloroform/n-Heptan/Aethanol (10:10:1) als Eluierungsmittel chromatographiert. Die einheitlichen Fraktionen werden vereinigt und eingedampft. Nach dem Anreiben des Rückstandes mit Aether erhält man 1,8 g (47%) 2,3,6-Trimethyl-2H-1,2-thiazin-5(6H)-on-1,1-dioxid in Form von farblosen Kristallen, Smp. 73-75°.

In eine Lösung von 2,5 g 2,3,6-Trimethyl-2H-1,2-thiazin-5(6H)-on-1,1-dioxid in 25 ml Methanol wird während 3 Stunden bei 20-25° ein mässig rascher Strom von trockenem Ammoniak eingeleitet, und hierauf wird das Gemisch über Nacht stehen gelassen. Die erhaltene dunkelgelbe Lösung wird unter vermindertem Druck zur Trockene eingeengt, und der kristalline Rückstand aus Aethanol umkristallisiert. Man erhält 2,3 g (85%) 4-Amino-N,1-dimethyl-2-oxo-3-penten- sulfonamid in Form von farblosen Kristallen, Smp. 140-143°.

## Beispiel 12

Nach 8-stündigem Kochen von 2,8 g (0,01 Mol) 3-Nitrobenzylidenacetessigsäureisopropylester und 2,34 g (0,01 Mol) 4-Amino-N-isopropyl-1-methyl-2-oxo-3-pentensulfonamid in einer Mischung von 10 ml Isopropanol und 5 ml Dimethylformamid werden 1,4 g (28%) 1,4-Dihydro-2,6-dimethyl-5-[2-(isopropylsulfamoyl)propionyl]-4-(3-nitrophenyl)nicotinsäureisopropylester erhalten, Smp. 155-158° (gelbes Kristallpulver aus Aether, nur eines der beiden möglichen Racemate).

Das als Ausgangsmaterial verwendete 4-Amino-N-isopropyl--1-methyl-2-oxo-3-pentensulfonamid kann wie in Beispiel 11 angegeben aus 2-Isopropyl-3-methyl-1,2-thiazin-5(6H)--on-1,1-dioxid hergestellt werden. Man erhält zunächst das 2-Isopropyl-3,6-dimethyl-2H-1,2-thiazin-5(6H)-on-1,1-dioxid, Smp. 61-65° (aus Aether), welches danach in das 4-Amino-N--isopropyl-1-methyl-2-oxo-3-pentensulfonamid, Smp. 117-120° (aus Aethanol) überführt wird.

## Beispiel 13

Nach 16-stündigem Kochen von 2,8 g (0,01 Mol) 3-Nitrobenzylidenacetessigsäureisopropylester und 2,34 g (0,01 Mol) N-Isopropyl-4-methylamino-2-oxo-3-pentansulfon- amid in einer Mischung von 25 ml Isopropanol und 12 ml Dimethylformamid werden 1,2 g (23%) 1,4-Dihydro-5-[(isopropylsulfamoyl)acetyl]--1,2,6-trimethyl-4-(3-nitrophenyl)nicotinsäureisopropylester vom Schmelzpunkt 67-69° (gelbes Kristallpulver aus Aether) erhalten. Auf Grund der Mikroanalyse und der NMR-Daten enthält dieses Produkt 0,5 Mol Aether.

Das als Ausgangsmaterial verwendete N-Isopropyl-4--methylamino-2-oxo-3-pentensulfonamid kann wie folgt hergestellt werden:

In eine Suspension von 4 g 2-Isopropyl-3-methyl-1,2--thiazin-5(6H)-on-1,1-dioxid in 40 ml Methanol wird während 1 Stunde bei 20-25° ein langsamer Strom von trockenem Methylamin eingeleitet, wobei nach ca. 30 Minuten eine klare gelbe Lösung entsteht. Hierauf wird 5 Stunden bei Raumtemperatur weiter gerührt, unter vermindertem Druck zur Trockene eingeengt, und der kristalline Rückstand aus Isopropanol umkristallisiert. Man erhält 3,8 g (82%) N-Isopropyl-4-methylamino-2-oxo-3-pentensulfonamid in Form von farblosen Kristallen, Smp. 83-85°.

Beispiel 14

Eine Lösung von 3,4 g (0,01 Mol) (±)-3-Nitrobenzylidenacetessigsäure-1-phenyläthylester in einer Mischung von 10 ml Isopropanol und 5 ml Dimethylformamid wird unter Argon mit 2,20 g (0,01 Mol) 4-Amino-N-isopropyl-2-oxo-3-pentensulfonamid versetzt und hierauf 8 Stunden zum Rückfluss erhitzt. Nach dem Einengen unter vermindertem Druck wird der ölige Rückstand an 300 g Kieselgel mit Methylenchlorid/Essigester (4:1) als Eluierungsmittel chromatographiert. Die homogenen Fraktionen liefern ein Oel (3,1 g), welches auf Grund der NMR-Daten aus einem Gemisch der beiden möglichen Racemate des 1,4-Dihydro-5-[(isopropylsulfamoyl)acetyl]-2,6-dimethyl-4-(3--nitrophenyl)nicotinsäure-1-phenyläthylesters besteht.

Zur Trennung der beiden Racemate wird das ölige Rohprodukt (3,1 g) in 100 ml Aether aufgenommen, und die erhaltene Lösung über Nacht bei Raumtemperatur stehen gelassen. Das auskristallisierte Produkt wird abfiltriert, mit wenig Aether gewaschen und aus Isopropanol umkristallisiert, wobei man 0,70 g (13%) des einen, reinen Racemates (Racemat A) in Form von gelben Kristallen erhält, Smp. 164-166°. Die ätherische Mutterlauge wird auf ca. 70 ml eingeengt, über Nacht bei Raumtemperatur stehen gelassen und von einer kleinen Menge des Racematengemisches abfiltriert (ca. 0,2 g). Das erhaltene Filtrat wird nun auf ca. 50 ml eingeengt und wiederum über

Nacht bei Raumtemperatur stehen gelassen. Der neue Niederschlag liefert nach Umkristallisation aus Isopropanol 0,50 g (9%) des anderen, reinen Racemates (Racemat B) in Form von gelben Kristallen, Smp. 155-157°.

Beispiel 15

Eine Lösung von 3,75 g (0,01 Mol) (±)-3-Nitrobenzylidenacetessigsäure-1-p-chlorphenyläthylesterund 2,20 g (0,01 Mol) 4-Amino-N-isopropyl-2-oxo-3-pentensulfonamid in einer Mischung von 10 ml Isopropanol und 5 ml Dimethylformamid wird 8 Stunden zum Rückfluss erhitzt und danach unter vermindertem Druck zur Trockene eingeengt. Das verbleibende Oel wird an 500 g Kieselgel mit Methylenchlorid/Essigester (4:1) als Eluierungsmittel chromatographiert, wobei die beiden gebildeten Racemate des 1,4-Dihydro-5-[(isopropylsulfamoyl)acetyl]--2,6-dimethyl-4-(3-nitrophenyl)nicotinsäure-1-p-chlorphenyläthylesters getrennt werden. Die ersten eluierten homogenen Fraktionen ergeben nach Eindampfen, Anreiben des Rückstandes mit Aether und Umkristallisation aus Aethanol 0,70 g (12%) des einen, reinen Racemates in Form von gelben Kristallen, Smp. 155-158°. Die folgenden eluierten homogenen Fraktionen liefern nach gleicher Behandlung 0,85 g (15%) des zweiten, reinen Racemates in Form von gelben Kristallen, Smp. 148-151°.

Beispiel 16

Eine Lösung von 6,8 g (0,02 Mol) (S)-3-Nitrobenzylidenacetessigsäure-1-phenyläthylester in einer Mischung von 20 ml Isopropanol und 10 ml Dimethylformamid wird unter Argon mit 4,40 g (0,02 Mol) 4-Amino-N-isopropyl-2-oxo-3-pentensulfonamid versetzt und hierauf 8 Stunden zum Rückfluss erhitzt. Nach dem Einengen unter vermindertem Druck wird der ölige Rückstand an 800 g Kieselgel mit Methylenchlorid/Essigester (4:1) als Eluierungsmittel chromatographiert, wobei die zwei erwarteten Epimeren als Gemisch eluiert werden. Die homogenen Fraktionen liefern ein Oel, das man in 300 ml Aether aufnimmt und über

Nacht bei Raumtemperatur stehen lässt. Das auskristallisierte Produkt wird abfiltriert, mit wenig Aether gewaschen und aus Aethanol umkristallisiert. Man erhält 2,3 g (21%) (R)-1,4--Dihydro-5-[(isopropylsulfamoyl)acetyl]-2,6-dimethyl-4-(3--nitrophenyl)nicotinsäure-(S)-1-phenyläthylester in Form von gelben Kristallen, Smp. 151-153°, spezifische Drehung: $[\alpha]_{546}^{20}$ = + 518° (c = 1,0% w/v, Aethanol). Auf Grund des NMR-Spektrums handelt es sich um das (+)-Enantiomer des in Beispiel 14 beschriebenen Racemates A.

Die erhaltene ätherische Mutterlauge wird auf 200 ml eingeengt und 2-3 Tage bei Raumtemperatur stehen gelassen, wobei das zweite gebildete Epimer langsam kristallisiert. Nach Umkristallisation aus Aethanol erhält man 1,75 g (16%) (S)-1,4--Dihydro-5-[(isopropylsulfamoyl)acetyl]-2,6-dimethyl-4-(3--nitrophenyl)nicotinsäure-(S)-1-phenyläthylesterin Form von gelben Kristallen, Smp. 128-131°, spezifische Drehung : $[\alpha]_{546}^{20}$ = - 163° (c = 1,0% w/v, Aethanol). Auf Grund des NMR-Spektrums handelt es sich um das (-)-Enantiomer des in Beispiel 14 beschriebenen Racemates B. Die angegebene (S)-Konfiguration für das chirale Zentrum in Position 4 wurde durch eine Röntgen-Strukturanalyse bestimmt.

## Beispiel 17

Gemäss der in Beispiel 16 beschriebenen Arbeitsweise werden aus (R)-3-Nitrobenzylidenacetessigsäure-1-phenyläthylester und 4-Amino-N-isopropyl-2-oxo-3-pentensulfonamid die zwei folgenden Epimeren erhalten:

(S)-1,4-Dihydro-5-[(isopropylsulfamoyl)acetyl]-2,6--dimethyl-4-(3-nitrophenyl)nicotinsäure-(R)-1-phenyläthylester in Form von gelben Kristallen, Smp. 151-153° (aus Aethanol), spezifische Drehung: $[\alpha]_{546}^{20}$ = -512° (c = 1,0% w/v, Aethanol). Auf Grund der NMR-Spektrums handelt es sich um das (-)-Enantiomer des in Beispiel 14 beschriebenen

Racemates A.

(R)-1,4-Dihydro-5-[(isopropylsulfamoyl)acetyl]-2,6-dimethyl-4-(3-nitrophenyl)nicotinsäure-(R)-1-phenyläthylester-in Form von gelben Kristallen, Smp. 128-131° (aus Aethanol), spezifische Drehung: $[\alpha]^{20}_{546}$ = + 165° (c = 1,0% w/v, Aethanol). Auf Grund des NMR-Spektrums handelt es sich um das (+)-Enantiomer des in Beispiel 14 beschriebenen Racemates B.

## Beispiel 18

Eine Lösung von 2,35 g (5 mMol) 1,4-Dihydro-5-[(isopropylsulfamoyl)acetyl]-2,6-dimethyl-4-(3-nitrophenyl)nicotinsäureäthylester in einer Mischung von 25 ml Aethanol und 5 ml Dimethylformamid wird unter Argon bei 15-20° mit 0,20 g (5 mMol) Natriumborhydrid portionenweise versetzt und 20 Stunden bei Raumtemperatur weiter gerührt. Hierauf wird die erhaltene Suspension vorsichtig mit 1N Salzsäure auf pH 4-5 gestellt, und das Aethanol unter vermindertem Druck abgedampft. Die verbleibende Lösung wird mit ungefähr dem doppelten Volumen Eis versetzt und mit Methylenchlorid extrahiert. Die organische Phase wird mit Wasser gewaschen, über Natriumsulfat getrocknet und zur Trockene eingeengt. Das verbleibende Oel wird an 600 g Kieselgel mit Methylenchlorid/Essigester (4:1) als Eluierungsmittel chromatographiert. Die einheitlichen Fraktionen werden vereinigt und eingedampft. Nach dem Anreiben des Rückstandes mit Aether erhält man 1,9 g (85%) 1,4-Dihydro-5-[(E)-2-(isopropylsulfamoyl)vinyl]-2,6-dimethyl-4-(3-nitrophenyl)nicotinsäureäthylester in Form von gelben Kristallen, Smp. 191-193°. Umkristallisation aus Aethanol erhöht den Schmelzpunkt nicht.

## Beispiel 19

Analog Beispiel 18 werden 1,86 g (4 mMol) 5-[(Aethylsulfamoyl)acetyl]-1,4-dihydro-2,6-dimethyl-4-(3-nitrophenyl)nicotinsäureisopropylester mit 0,16 g (4 mMol)

Natriumborhydrid in einer Mischung von 20 ml Isopropanol und 4 ml Dimethylformamid versetzt. Nach Chromatographie mit Methylenchlorid/Essigester (4:1) als Eluierungsmittel erhält man 1,5 g (83%) 5-[(E)-2-(Aethylsulfamoyl)vinyl]-1,4-dihydro-2,6-dimethyl-4-(3-nitrophenyl)nicotinsäureisopropylester in Form von gelben Kristallen, Smp. 187-189°. Umkristallisation aus Methylenchlorid/Aether erhöht den Schmelzpunkt nicht.

## Beispiel 20

Analog Beispiel 18 wird eine Lösung von 1,56 g (3 mMol) 1,4-Dihydro-5-[(isopropylsulfamoyl)acetyl]-2,6-dimethyl-4-(3-nitrophenyl)nicotinsäure-2,2,2-trifluoräthylester in 15 ml Isopropanol mit 0,12 g (3 mMol) Natriumborhydrid versetzt. Nach Chromatographie mit Methylenchlorid/Essigester (4:1) als Eluierungsmittel erhält man 1,25 g (83%) 1,4-Dihydro-5-[(E)-2-(isopropylsulfamoyl)vinyl]-2,6-dimethyl-4-(3-nitrophenyl)nicotinsäure-2,2,2-trifluoräthylester in Form von gelben Kristallen, Smp. 180-182°. Umkristallisation aus Isopropanol erhöht den Schmelzpunkt nicht.

## Beispiel 21

Analog Beispiel 18 werden 1,02 g (2 mMol) 4-(2-Chlor-5-nitrophenyl)-1,4-dihydro-5-[(isopropylsulfamoyl)acetyl]-2,6-dimethylnicotinsäureisopropylester mit 0,08 g (2 mMol) Natriumborhydrid in einer Mischung von 10 ml Isopropanol und 2 ml 1,2-Dimethoxyäthan versetzt. Nach Chromatographie mit Methylenchlorid/Essigester (4:1) als Eluierungsmittel erhält man 0,70 g (70%) 4-(2-Chlor-5-nitrophenyl)-1,4-dihydro-5-[(E)-2-(isopropylsulfamoyl)vinyl]-2,6-dimethylnicotinsäureisopropylester in Form von gelben Kristallen, Smp. 199-201°.

Beispiel 22

Analog Beispiel 18 werden 1,05 g (2 mMol) 1,4-Dihydro--5-[(isopropylsulfamoyl)acetyl]-2,6-dimethyl-4-(3-nitrophenyl)-nicotinsäure-2-propoxyäthylester mit 0,08 g (2 mMol) Natriumborhydrid in einer Mischung von 10 ml Aethanol und 2 ml Dimethylformamid versetzt. Nach Chromatographie mit Methylenchlorid/Essigester (4:1) als Eluierungsmittel erhält man 0,90 g (88%) 1,4-Dihydro-5-[(E)-2-(isopropylsulfamoyl)vinyl]--2,6-dimethyl-4-(3-nitrophenyl)nicotinsäure-2-propoxyäthylester in Form von gelben Kristallen, Smp. 152-154°.

Beispiel 23

Analog Beispiel 18 werden 1,03 g (2 mMol) 5-[(Benzylmethylsulfamoyl)acetyl]-1,4-dihydro-2,6-dimethyl-4-(3--nitrophenyl)nicotinsäuremethylester mit 0,08 g (2 mMol) Natriumborhydrid in einer Mischung von 10 ml Isopropanol und 4 ml Dimethylformamid versetzt. Nach Chromatographie mit Methylenchlorid/Essigester (4:1) als Eluierungsmittel erhält man 0,64 g (64%) 5-[(E)-2-(Benzylmethylsulfamoyl)-vinyl]-1,4-dihydro-2,6-dimethyl-4-(3-nitrophenyl)nicotinsäuremethylester in Form von gelben Kristallen, Smp. 208-210°.

Beispiel 24

Analog Beispiel 18 werden 1,08 g (2 mMol) (S)-1,4--Dihydro-5-[(isopropylsulfamoyl)acetyl]-2,6-dimethyl-4-(3--nitrophenyl)nicotinsäure-(R)-1-phenyläthylester mit 0,08 g (2 mMol) Natriumborhydrid in einer Mischung von 10 ml Isopropanol und 2 ml Dimethylformamid versetzt. Das Produkt wird mit Methylenchlorid/Essigester (4:1) als Eluierungsmittel chromatographiert und dann aus Aethanol umkristallisiert. Man erhält 0,82 g (78%) (S)-1,4-Dihydro-5-[(E)-2-(isopropylsulfamoyl)vinyl]-2,6-dimethyl-4-(3-nitrophenyl)nicotinsäure--(R)-1-phenyläthylester in Form von gelben Kristallen, Smp. 198-201°, spezifische Drehung: $[\alpha]_{546}^{20}$ = - 290° (c =

1,0% w/v, Chloroform).

Beispiel 25

Eine Lösung von 4,12 g (0,02 Mol) N-Aethyl-4-amino-2--oxo-3-pentensulfonamid, 2,81 g (0,02 Mol) 2-Chlorbenzaldehyd und 3,76 g (0,02 Mol) 4-Acetoxyacetessigsäureäthylester in einer Mischung von 20 ml Aethanol und 10 ml Dimethylformamid wird unter Argon 15 Stunden zum Rückfluss erhitzt. Die Lösung wird dann unter vermindertem Druck zur Trockene eingeengt, und das verbleibende Oel an 360 g Kieselgel mit Methylenchlorid/-Essigester (4:1) als Eluierungsmittel chromatographiert. Die homogenen Fraktionen werden vereinigt und eingedampft. Das verbleibende Oel kristallisiert beim Anreiben mit Aether. Man erhält 4,0 g (40%) 2-Acetoxymethyl-5-[(äthylsulfamoyl)-acetyl]-4-(2-chlorphenyl)-1,4-dihydro-6-methylnicotinsäure-äthylester in Form von gelben Kristallen, Smp. 106-108°. Umkristallisation aus Aethanol liefert ein Produkt vom Schmelzpunkt 109-110°.

Beispiel 26

Eine Lösung von 3,0 g (6 mMol) 1,4-Dihydro-5-[(isopropylsulfamoyl)acetyl]-2,6-dimethyl-4-(3-nitrophenyl)nicotinsäure-2-chloräthylester in 18 ml Dimethylformamid wird unter Argon mit 0,75 g (9 mMol) trockenem Natriumacetat versetzt und hierauf 1 Stunde zum Rückfluss erhitzt. Das Gemisch wird dann unter vermindertem Druck zur Trockene eingeengt, und der Rückstand zwischen Wasser und Methylenchlorid verteilt. Die organische Phase wird mit einer gesättigten wässrigen Lösung von Natriumchlorid gewaschen, über Natriumsulfat getrocknet und zur Trockene eingedampft. Das verbleibende Oel wird an 300 g Kieselgel mit Methylenchlorid/Essigester (4:1) als Eluierungsmittel chromatographiert. Die einheitlichen Fraktionen werden vereinigt und eingedampft. Nach dem Anreiben des Rückstandes mit Aether erhält man 1,85 g (59%) 1,4-Dihydro-5-[(isopropylsulfamoyl)acetyl]-2,6-dimethyl-4-(3-nitrophenyl)nicotinsäure--2-acetoxyäthylester in Form von hellgelben Kristallen, Smp. 133-136°. Umkristallisation aus Isopropanol liefert ein Produkt vom Schmelzpunkt 134-137°.

## Beispiel 27

Eine Suspension von 2,09 g (4 mMol) 1,4-Dihydro-5--[(isopropylsulfamoyl)acetyl]-2,6-dimethyl-4-(3-nitrophenyl)nicotinsäure-2-acetoxyäthylester in 20 ml Aethanol wird bei Raumtemperatur tropfenweise mit einer Lösung von 0,36 g (9 mMol) Natriumhydroxid in 24 ml Aethanol versetzt. Die erhaltene dunkelorange Lösung wird anschliessend 1 Stunde bei Raumtemperatur weitergerührt. Das Aethanol wird hierauf bei 25-30° unter vermindertem Druck abgedampft, und der Rückstand in 20 ml Wasser gelöst. Man säuert vorsichtig mit 2N Salzsäure an, extrahiert mehrmals mit einem Gemisch Methylenchlorid/-Essigester (9:1) und trocknet den Extrakt über Natriumsulfat. Nach dem Eindampfen des Lösungsmittels wird der feste Rückstand aus Aethanol umkristallisiert. Man erhält 1,55 g (80%) 1,4-Dihydro-5-[(isopropylsulfamoyl)acetyl]-2,6-dimethyl-4--(3-nitrophenyl)nicotinsäure-2-hydroxyäthylester in Form von hellgelben Kristallen, Smp. 185-188°.

## Beispiel 28

In analoger Weise wie in Beispiel 4 beschrieben können die folgenden Verbindungen hergestellt werden:

1,4-Dihydro-5-[(isopropylsulfamoyl)acetyl]-2,6-dimethyl-4-(3-nitrophenyl)nicotinsäurecyclopentylester, Smp. 112-115°, aus 3-Nitrobenzylidenacetessigsäurecyclopentylester und 4-Amino-N-isopropyl-2-oxo-3-pentensulfonamid;

1,4-Dihydro-5-[(isopropylsulfamoyl)acetyl]-2,6-dimethyl-4-(3-nitrophenyl)nicotinsäurecyclopropylmethylester, Smp. 152-154°, aus 3-Nitrobenzylidenacetessigsäurecyclopropylmethylester und 4-Amino-N-isopropyl-2-oxo-3-pentensulfonamid und

1,4-Dihydro-5-[(isopropylsulfamoyl)acetyl]-2,6-dimethyl-4-(3-nitrophenyl)nicotinsäure-2-methylthioäthylester, Smp. 121-124°, aus 3-Nitrobenzylidenacetessigsäure-2-methylthioäthylester und 4-Amino-N-isopropyl-2-oxo-3-pentensulfonamid.

## Beispiel A

Herstellung von Tabletten folgender Zusammensetzung:

| | | |
|---|---|---|
| I. | 1,4-Dihydro-5-[(isopropylsulfamoyl)-acetyl]-2,6-dimethyl-4-(3-nitrophenyl)-nicotinsäure-2-propoxyäthylester mikronisiert | 20,0 mg |
| | Milchzucker pulv. | 40,0 mg |
| | Maisstärke weiss | 24,9 mg |
| II. | Dioctylnatriumsulfosuccinat | 0,1 mg |
| | Maisstärke weiss | 5,0 mg |
| | Wasser | q.s. |
| III. | Maisstärke weiss | 6,0 mg |
| IV. | Talk | 3,0 mg |
| | Magnesiumstearat | 1,0 mg |
| | | 100,0 mg |

Die Stoffe der Phase I werden gesiebt und gemischt. Diese Mischung wird mit dem Maisstärkekleister II befeuchtet und geknetet. Die feuchte Masse wird granuliert, getrocknet und auf eine geeignete Korngrösse zugerüstet. Phase III wird beigemischt. Diese Mischung wird noch während kurzer Zeit mit Phase IV gemischt.

Die pressfertige Mischung wird zu Tabletten à 100 mg mit Bruchrille verpresst.

## Beispiel B

Herstellung von Tabletten folgender Zusammensetzung:

| | | |
|---|---|---|
| I. | 4-(2-Chlor-5-nitrophenyl)-1,4-dihydro-5-[isopropylsulfamoyl)acetyl]-2,6-dimethyl-nicotinsäureisopropylester | 200,0 mg |
| | Milchzucker pulv. | 42,9 mg |
| | Maisstärke weiss | 50,0 mg |
| II. | Dioctylnatriumsulfosuccinat | 0,1 mg |
| | Maisstärke weiss | 20,0 mg |
| | Wasser | q.s. |
| III. | Maisstärke weiss | 30,0 mg |
| IV. | Talk | 3,5 mg |
| | Magnesiumstearat | 3,5 mg |
| | | 350,0 mg |

Die Stoffe der Phase I werden gesiebt und gemischt. Diese Mischung wird mit dem Maisstärkekleister II befeuchtet und geknetet. Die feuchte Masse wird granuliert, getrocknet und auf eine geeignete Korngrösse zugerüstet. Phase III wird beigemischt. Diese Mischung wird noch während kurzer Zeit mit Phase IV gemischt.

Die pressfertige Masse wird zu Tabletten à 350 mg mit Bruchrille verpresst.

## Beispiel C

Herstellung von Kapseln folgender Zusammensetzung:

| | | |
|---|---|---|
| I. | 1,4-Dihydro-5-[(isopropylsulfamoyl)acetyl]-2,6-dimethyl-4-(3-nitrophenyl)nicotinsäure-2-propoxyäthylester mikronisiert | 20,0 mg |
| | Milchzucker pulv. | 48,0 mg |
| II. | Maisstärke | 5,0 mg |
| | Wasser | q.s. |
| III. | Milchzucker krist. | 50,0 mg |
| | Maisstärke | 15,0 mg |
| IV. | Talk | 10,0 mg |
| | Magnesiumstearat | 2,0 mg |
| | | 150,0 mg |

Die Stoffe der Phase I werden gesiebt und gemischt. Diese Mischung wird mit dem Maisstärkekleister II befeuchtet und geknetet. Die feuchte Masse wird granuliert, getrocknet und auf eine geeignete Korngrösse zugerüstet. Phase III wird beigemischt. Diese Mischung wird noch während kurzer Zeit mit Phase IV gemischt.

Die Kapselmischung wird zu je 150 mg in Kapseln Grösse 2 gefüllt.

## Beispiel D

Man stellt eine wässrige Tropfensuspension folgender Zusammensetzung her:

| | | 5 mg pro 1 ml |
|---|---|---|
| 1,4-Dihydro-5-[isopropylsulfamoyl)-acetyl]-2,6-dimethyl-4-(3-nitrophenyl)-nicotinsäure-2-propoxyäthylester | | 0,05 g |
| Natriumbenzoat | | 0,035 g |
| Saccharin-Natrium | | 0,015 g |
| Acrylsäurepolymerisat | 0,1 - | 1,0 g |
| Saccharose | | 3,5 g |
| Citronensäure | | 0,025 g |
| Polyoxyäthylen-stearat | 0,002 - | 0,01 g |
| Natriumhydroxid | | q.s. |
| Aroma | | q.s. |
| Lebensmittelfarbstoff | | q.s. |
| Wasser entsalzt | ad | 10,0 ml |

## Beispiel E

Wenn man nach den in den Beispielen A-D beschriebenen Verfahren arbeitet, können aus den folgenden, ebenfalls bevorzugten Verbindungen Tabletten, Kapseln und Injektionspräparate hergestellt werden:

1,4-Dihydro-5-[(isopropylsulfamoyl)acetyl]-2,6-dimethyl-4-(3-nitrophenyl)nicotinsäureisopropylester,

4-(2,5-Dichlorphenyl)-1,4-dihydro-5-[(isopropylsulfamoyl)acetyl]-2,6-dimethylnicotinsäureisopropylester und

(S)-1,4-Dihydro-5-[(isopropylsulfamoyl)acetyl]-2,6-dimethyl-4-(3-nitrophenyl)nicotinsäure-(S)-1-phenyläthylester.

## Patentansprüche

1. Dihydropyridinderivate der allgemeinen Formel

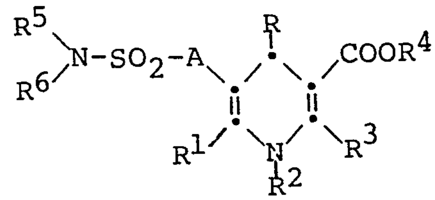

I

worin A die Gruppe $-CH(R^7)-CO-$ oder $-CH\overset{(E)}{=}CH-$, R Aryl oder einen heterocyclischen Rest mit bis zu drei Heteroatomen, wobei als Heteroatome Sauerstoff, Stickstoff oder Schwefel in Frage kommen, $R^1$ Wasserstoff oder Methyl, $R^2$ Wasserstoff oder $C_1$-$C_4$-Alkyl, $R^3$ $C_1$-$C_4$-Alkyl oder $C_1$-$C_4$-Alkanoyloxymethyl, $R^4$ $C_1$-$C_6$-Alkyl, $C_3$-$C_6$-Alkenyl, $C_3$-$C_6$-Alkinyl, $C_3$-$C_6$-Cycloalkyl, $C_3$-$C_6$-Cycloalkyl-$C_1$-$C_6$-alkyl, Cyan-$C_2$-$C_6$-alkyl, Halogen-$C_2$-$C_6$-alkyl, Hydroxy-$C_2$-$C_6$-alkyl, $\omega,\omega,\omega$-Trifluor-$C_1$-$C_6$-alkyl, $C_1$-$C_6$-Alkoxy-$C_1$-$C_6$-alkyl, $C_1$-$C_6$-Alkoxy-$C_1$-$C_6$-alkoxy-$C_1$-$C_6$-alkyl, $C_1$-$C_4$-Alkanoyloxy-$C_1$-$C_6$-alkyl, $C_3$-$C_6$-Alkenyloxy-$C_1$-$C_6$-alkyl, $C_1$-$C_6$-Alkylthio-$C_1$-$C_6$-alkyl, Benzyloxy-$C_1$-$C_6$-alkyl, gegebenenfalls durch Halogen, Cyan, Di-$C_1$-$C_6$-alkylamino, $C_1$-$C_6$-Alkoxy, $C_1$-$C_6$-Alkyl, Trifluormethyl oder Nitro substituiertes Phenyl oder gegebenenfalls durch Halogen, Cyan, Di-$C_1$-$C_6$-alkylamino, $C_1$-$C_6$-Alkoxy, $C_1$-$C_6$-Alkyl, Trifluormethyl oder Nitro substituiertes Phenyl-$C_1$-$C_6$-alkyl, $R^5$ $C_1$-$C_6$-Alkyl oder $C_3$-$C_6$-Cycloalkyl, $R^6$ Wasserstoff, $C_1$-$C_6$-Alkyl, $C_3$-$C_6$-Alkenyl, $C_3$-$C_6$-Alkinyl, $C_3$-$C_6$-Cycloalkyl, $C_3$-$C_6$-Cycloalkyl-$C_1$-$C_6$-alkyl oder gegebenenfalls durch $C_1$-$C_6$-Alkyl, $C_1$-$C_6$-Alkoxy oder Halogen substituiertes Phenyl-$C_1$-$C_6$-alkyl und $R^7$ Wasserstoff, $C_1$-$C_6$-Alkyl oder gegebenenfalls durch $C_1$-$C_6$-Alkyl,

$C_1$-$C_6$-Alkoxy oder Halogen substituiertes Phenyl-$C_1$-$C_6$-alkyl oder $R^6$ und $R^7$ zusammen eine $-(CH_2)_n$-Gruppe, worin n die Zahl 2 oder 3 bedeutet, bedeuten,

in Form von Isomeren, Isomerengemischen, Racematen und optischen Antipoden.

2. Verbindungen gemäss Anspruch 1, worin $R^4$ $C_1$-$C_6$-Alkyl, Cyan-$C_2$-$C_6$-alkyl, Halogen-$C_2$-$C_6$-alkyl, Hydroxy-$C_2$-$C_6$-alkyl, ω,ω,ω-Trifluor-$C_1$-$C_6$-alkyl, $C_1$-$C_6$-Alkoxy-$C_1$-$C_6$-alkyl, $C_1$-$C_4$-Alkanoyloxy-$C_1$-$C_6$-alkyl, Benzyloxy-$C_1$-$C_6$-alkyl oder gegebenenfalls durch Halogen substituiertes Phenyl-$C_1$-$C_6$-alkyl, $R^5$ $C_1$-$C_6$-Alkyl, $R^6$ Wasserstoff, $C_1$-$C_6$-Alkyl, $C_3$-$C_6$-Alkenyl oder Phenyl-$C_1$-$C_6$-alkyl, $R^7$ Wasserstoff oder $C_1$-$C_6$-Alkyl oder $R^6$ und $R^7$ zusammen eine $-(CH_2)_n$-Gruppe, worin n die Zahl 2 oder 3 bedeutet, und R Naphthyl, gegebenenfalls durch $C_1$-$C_6$-Alkyl, Halogen, Trifluormethyl oder Nitro monosubstituiertes oder durch Halogen bzw. Halogen und Nitro disubstituiertes Phenyl, Imidazolyl oder Pyridyl bedeuten.

3. Verbindungen gemäss Anspruch 1 oder 2, worin A die Gruppe $-CH(R^7)-CO-$ bedeutet.

4. Verbindungen gemäss einem der Ansprüche 1-3, worin R 3-Nitrophenyl, 2-Chlor-5-nitrophenyl oder 2,5-Dichlorphenyl, $R^1$ Methyl, $R^2$ Wasserstoff und $R^3$ $C_1$-$C_4$-Alkyl, vorzugsweise Methyl, bedeuten.

5. Verbindungen gemäss einem der Ansprüche 1-4, worin $R^4$ $C_1$-$C_6$-Alkyl, Cyan-$C_2$-$C_6$-alkyl, Halogen-$C_2$-$C_6$-alkyl, Hydroxy-$C_2$-$C_6$-alkyl, ω,ω,ω-Trifluor-$C_1$-$C_6$-alkyl, $C_1$-$C_6$-Alkoxy-$C_1$-$C_6$-alkyl, $C_1$-$C_4$-Alkanoyloxy-$C_1$-$C_6$-alkyl, Benzyloxy-$C_1$-$C_6$-alkyl oder gegebenenfalls durch Halogen substituiertes Phenyl-$C_1$-$C_6$-alkyl, vorzugsweise $C_1$-$C_6$-Alkyl, ω,ω,ω-Trifluor-

$C_1$-$C_6$-alkyl, $C_1$-$C_6$-Alkoxy-$C_1$-$C_6$-alkyl oder Phenyl-$C_1$-$C_6$-alkyl, ganz besonders bevorzugt Isopropyl, 2,2,2-Trifluoräthyl, 2-Propoxyäthyl oder 1-Phenyläthyl, $R^5$ $C_1$-$C_6$-Alkyl, vorzugsweise Methyl, Aethyl oder Isopropyl, und $R^6$ und $R^7$ je Wasserstoff bedeuten.

6. Verbindungen gemäss einem der Ansprüche 1-5, worin A die Gruppe -CH($R^7$)-CO-, R 3-Nitrophenyl, 2-Chlor-5--nitrophenyl oder 2,5-Dichlorphenyl, $R^1$ und $R^3$ je Methyl, $R^2$, $R^6$ und $R^7$ je Wasserstoff, $R^4$ Isopropyl, 2,2,2-Trifluoräthyl, 2-Propoxyäthyl oder 1-Phenyläthyl und $R^5$ Methyl, Aethyl oder Isopropyl bedeuten.

7. 5-[(Aethylsulfamoyl)acetyl]-1,4-dihydro-2,6--dimethyl-4-(3-nitrophenyl)nicotinsäureisopropylester.

8. 1,4-Dihydro-2,6-dimethyl-5-[(methylsulfamoyl)-acetyl]-4-(3-nitrophenyl)nicotinsäureisopropylester.

9. 1,4-Dihydro-2,6-dimethyl-5-[(methylsulfamoyl)-acetyl]-4-(3-nitrophenyl)nicotinsäure-2,2,2-trifluoräthylester.

10. 4-(2-Chlor-5-nitrophenyl)-1,4-dihydro-5-[(isopropyl-sulfamoyl)acetyl]-2,6-dimethylnicotinsäureisopropylester.

11. 4-(2,5-Dichlorphenyl)-1,4-dihydro-5-[(isopropyl-sulfamoyl)acetyl]-2,6-dimethylnicotinsäureisopropylester.

12. 1,4-Dihydro-5-[(isopropylsulfamoyl)acetyl]-2,6-dimethyl-4-(3-nitrophenyl)nicotinsäure-2-propoxyäthylester.

13. (S)-1,4-Dihydro-5-[(isopropylsulfamoyl)acetyl]-2,6-dimethyl-4-(3-nitrophenyl)nicotinsäure-(S)-1-phenyläthylester.

14. Verbindungen der allgemeinen Formel

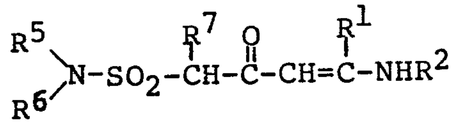

II

worin $R^1$ Wasserstoff oder Methyl, $R^2$ Wasserstoff oder $C_1$-$C_4$-Alkyl, $R^5$ $C_1$-$C_6$-Alkyl oder $C_3$--$C_6$-Cycloalkyl, $R^6$ Wasserstoff, $C_1$-$C_6$-Alkyl, $C_3$-$C_6$-Alkenyl, $C_3$-$C_6$-Alkinyl, $C_3$-$C_6$-Cycloalkyl, $C_3$-$C_6$-Cycloalkyl-$C_1$-$C_6$-alkyl oder gegebenenfalls durch $C_1$-$C_6$-Alkyl, $C_1$-$C_6$-Alkoxy oder Halogen substituiertes Phenyl-$C_1$-$C_6$-alkyl und $R^7$ Wasserstoff, $C_1$-$C_6$-Alkyl oder gegebenenfalls durch $C_1$-$C_6$-Alkyl, $C_1$-$C_6$-Alkoxy oder Halogen substituiertes Phenyl-$C_1$-$C_6$-alkyl oder $R^6$ und $R^7$ zusammen eine $-(CH_2)_n-$Gruppe, worin n die Zahl 2 oder 3 bedeutet, bedeuten.

15. Dihydropyridinderivate gemäss einem der Ansprüche 1-13 zur Anwendung als therapeutische Wirkstoffe.

16. Dihydropyridinderivate gemäss einem der Ansprüche 1-13 zur Anwendung bei der Bekämpfung bzw. Verhütung von Angina pectoris, Ischämie, Bluthochdruck und Migräne.

17. 1,4-Dihydro-5-[(isopropylsulfamoyl)acetyl]-2,6-dimethyl-4-(3-nitrophenyl)nicotinsäure-2-propoxyäthylester zur Anwendung als therapeutischer Wirkstoff.

18. 1,4-Dihydro-5-[(isopropylsulfamoyl)acetyl]-2,6-dimethyl-4-(3-nitrophenyl)nicotinsäure-2-propoxyäthylester zur Anwendung bei der Bekämpfung bzw. Verhütung von Angina pectoris, Ischämie, Bluthochdruck und Migräne.

19. Verfahren zur Herstellung einer Verbindung gemäss einem der Ansprüche 1-13, dadurch gekennzeichnet, dass man

a) zur Herstellung von Verbindungen der Formel I, worin A die Gruppe $-CH(R^7)-CO-$ bedeutet, d.h. von Verbindungen der allgemeinen Formel

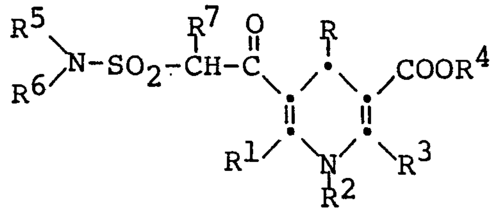

Ia

worin R, $R^1$, $R^2$, $R^3$, $R^4$, $R^5$, $R^6$ und $R^7$ die in Anspruch 1 angegebene Bedeutung besitzen,

ein Enamin der allgemeinen Formel

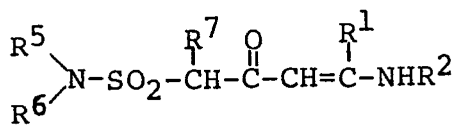

II

worin $R^1$, $R^2$, $R^5$, $R^6$ und $R^7$ die in Anspruch 1 angegebene Bedeutung besitzen,

mit einer Ylidenverbindung der allgemeinen Formel

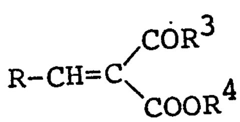

III

worin R, $R^3$ und $R^4$ die in Anspruch 1 angegebene Bedeutung besitzen,

umsetzt, oder

b) zur Herstellung von Verbindungen der Formel I, worin A die Gruppe $-CH\overset{(E)}{=}CH-$ bedeutet, d.h. von Verbindungen der allgemeinen Formel

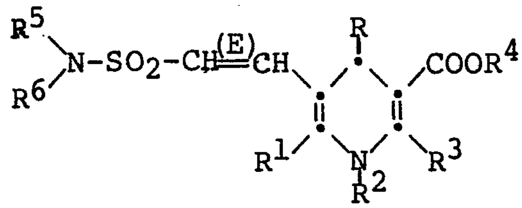

Ib

worin R, $R^1$, $R^2$, $R^3$, $R^4$, $R^5$, $R^6$ und $R^7$ die in Anspruch 1 angegebene Bedeutung besitzen, eine Verbindung der obigen Formel Ia, worin $R^7$ Wasserstoff bedeutet, mit Natriumborhydrid umsetzt, oder

c) erwünschtenfalls ein erhaltenes Isomerengemisch in die Isomeren auftrennt.

20. Arzneimittel, enthaltend ein Dihydropyridinderivat gemäss einem der Ansprüche 1-13 und ein therapeutisch inertes Excipiens.

21. Mittel zur Bekämpfung bzw. Verhütung von Angina pectoris, Ischämie, Bluthochdruck und Migräne, enthaltend ein Dihydropyridinderivat gemäss einem der Ansprüche 1-13 und ein therapeutisch inertes Excipiens.

22. Arzneimittel, enthaltend 1,4-Dihydro-5-[(isopropylsulfamoyl)acetyl]-2,6-dimethyl-4-(3-nitrophenyl)nicotinsäure-2-propoxyäthylester und ein therapeutisch inertes Excipiens.

23. Mittel zur Bekämpfung bzw. Verhütung von Angina pectoris, Ischämie, Bluthochdruck und Migräne, enthaltend 1,4-Dihydro-5-[(isopropylsulfamoyl)acetyl]-2,6-dimethyl-4-(3-nitrophenyl)nicotinsäure-2-propoxyäthylester und ein therapeutisch inertes Excipiens.

24. Verwendung eines Dihydropyridinderivates gemäss einem der Ansprüche 1-13 bei der Bekämpfung bzw. Verhütung

von Krankheiten.

25. Verwendung eines Dihydropyridinderivates gemäss einem der Ansprüche 1-13 bei der Bekämpfung bzw. Verhütung von Angina pectoris, Ischämie, Bluthochdruck und Migräne.

26. Verwendung eines Dihydropyridinderivates gemäss einem der Ansprüche 1-13 zur Herstellung eines Mittels gegen Angina pectoris, Ischämie, Bluthochdruck und/oder Migräne.

27. Verwendung von 1,4-Dihydro-5-[(isopropylsulfamoyl)-acetyl]-2,6-dimethyl-4-(3-nitrophenyl)nicotinsäure-2-propoxyäthylester nach einem der Ansprüche 24-26.

***

Patentansprüche für Oesterreich

1. Verfahren zur Herstellung von Dihydropyridinderivaten der allgemeinen Formel

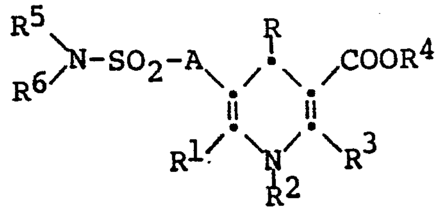

I

worin A die Gruppe $-CH(R^7)-CO-$ oder $-CH\overset{(E)}{=}CH-$, R Aryl oder einen heterocyclischen Rest mit bis zu drei Heteroatomen, wobei als Heteroatome Sauerstoff, Stickstoff oder Schwefel in Frage kommen, $R^1$ Wasserstoff oder Methyl, $R^2$ Wasserstoff oder $C_1$-$C_4$-Alkyl, $R^3$ $C_1$-$C_4$-Alkyl oder $C_1$-$C_4$-Alkanoyloxymethyl, $R^4$ $C_1$-$C_6$-Alkyl, $C_3$-$C_6$-Alkenyl, $C_3$-$C_6$-Alkinyl, $C_3$-$C_6$-Cycloalkyl, $C_3$-$C_6$-Cycloalkyl-$C_1$-$C_6$-alkyl, Cyan-$C_2$-$C_6$-alkyl, Halogen-$C_2$-$C_6$-alkyl, Hydroxy-$C_2$-$C_6$-alkyl, ω,ω,ω-Trifluor-$C_1$-$C_6$-alkyl, $C_1$-$C_6$-Alkoxy-$C_1$-$C_6$-alkyl, $C_1$-$C_6$-Alkoxy-$C_1$-$C_6$-alkoxy-$C_1$-$C_6$-alkyl, $C_1$-$C_4$-Alkanoyloxy-$C_1$-$C_6$-alkyl, $C_3$-$C_6$-Alkenyloxy-$C_1$-$C_6$-alkyl, $C_1$-$C_6$-Alkylthio-$C_1$-$C_6$-alkyl, Benzyloxy-$C_1$-$C_6$-alkyl, gegebenenfalls durch Halogen, Cyan, Di-$C_1$-$C_6$-alkylamino, $C_1$-$C_6$-Alkoxy, $C_1$-$C_6$-Alkyl, Trifluormethyl oder Nitro substituiertes Phenyl oder gegebenenfalls durch Halogen, Cyan, Di-$C_1$-$C_6$-alkylamino, $C_1$-$C_6$-Alkoxy, $C_1$-$C_6$-Alkyl, Trifluormethyl oder Nitro substituiertes Phenyl-$C_1$-$C_6$-alkyl, $R^5$ $C_1$-$C_6$-Alkyl oder $C_3$-$C_6$-Cycloalkyl, $R^6$ Wasserstoff, $C_1$-$C_6$-Alkyl, $C_3$-$C_6$-Alkenyl, $C_3$-$C_6$-Alkinyl, $C_3$-$C_6$-Cycloalkyl, $C_3$-$C_6$-Cycloalkyl-$C_1$-$C_6$-alkyl oder gegebenenfalls durch $C_1$-$C_6$-Alkyl, $C_1$-$C_6$-Alkoxy oder Halogen substituiertes Phenyl-$C_1$-$C_6$-alkyl und $R^7$ Wasserstoff, $C_1$-

$C_6$-Alkyl oder gegebenenfalls durch $C_1$-$C_6$-Alkyl, $C_1$-$C_6$-Alkoxy oder Halogen substituiertes Phenyl-$C_1$-$C_6$-alkyl oder $R^6$ und $R^7$ zusammen eine $-(CH_2)_n$-Gruppe, worin n die Zahl 2 oder 3 bedeutet, bedeuten,

in Form von Isomeren, Isomerengemischen, Racematen und optischen Antipoden, dadurch gekennzeichnet, dass man

a) zur Herstellung von Verbindungen der Formel I, worin A die Gruppe $-CH(R^7)-CO-$ bedeutet, d.h. von Verbindungen der allgemeinen Formel

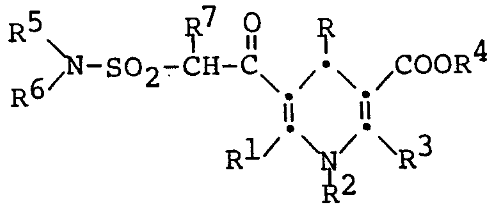

Ia

worin R, $R^1$, $R^2$, $R^3$, $R^4$, $R^5$, $R^6$ und $R^7$ die oben angegebene Bedeutung besitzen,

ein Enamin der allgemeinen Formel

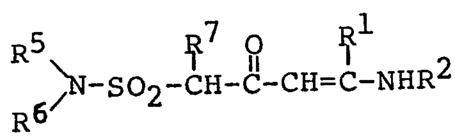

II

worin $R^1$, $R^2$, $R^5$, $R^6$ und $R^7$ die oben angegebene Bedeutung besitzen,

mit einer Ylidenverbindung der allgemeinen Formel

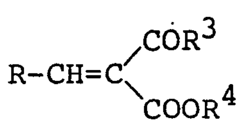

III

worin R, $R^3$ und $R^4$ die oben angegebene Bedeutung besitzen,

umsetzt, oder

b) zur Herstellung von Verbindungen der Formel I, worin A die Gruppe $-CH\overset{(E)}{=}CH-$ bedeutet, d.h. von Verbindungen der allgemeinen Formel

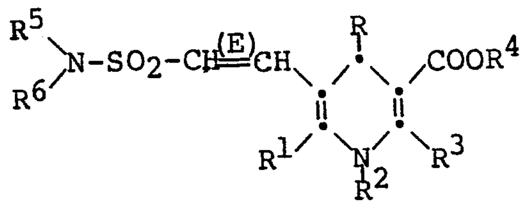

Ib

worin R, $R^1$, $R^2$, $R^3$, $R^4$, $R^5$, $R^6$ und $R^7$ die oben angegebene Bedeutung besitzen, eine Verbindung der obigen Formel Ia, worin $R^7$ Wasserstoff bedeutet, mit Natriumborhydrid umsetzt, oder

c) erwünschtenfalls ein erhaltenes Isomerengemisch in die Isomeren auftrennt.

2. Verfahren gemäss Anspruch 1, worin $R^4$ $C_1$-$C_6$-Alkyl, Cyan-$C_2$-$C_6$-alkyl, Halogen-$C_2$-$C_6$-alkyl, Hydroxy-$C_2$-$C_6$-alkyl, ω,ω,ω-Trifluor-$C_1$-$C_6$-alkyl, $C_1$-$C_6$-Alkoxy-$C_1$-$C_6$-alkyl, $C_1$-$C_4$-Alkanoyloxy-$C_1$-$C_6$-alkyl, Benzyloxy-$C_1$-$C_6$-alkyl oder gegebenenfalls durch Halogen substituiertes Phenyl-$C_1$-$C_6$-alkyl, $R^5$ $C_1$-$C_6$-Alkyl, $R^6$ Wasserstoff, $C_1$-$C_6$-Alkyl, $C_3$-$C_6$-Alkenyl oder Phenyl-$C_1$-$C_6$-alkyl, $R^7$ Wasserstoff oder $C_1$-$C_6$-Alkyl oder $R^6$ und $R^7$ zusammen eine $-(CH_2)_n$-Gruppe, worin n die Zahl 2 oder 3 bedeutet, und R Naphthyl, gegebenenfalls durch $C_1$-$C_6$-Alkyl, Halogen, Trifluormethyl oder Nitro monosubstituiertes oder durch Halogen bzw. Halogen und Nitro disubstituiertes Phenyl, Imidazolyl oder Pyridyl bedeuten.

3. Verfahren gemäss Anspruch 1 oder 2, worin A die Gruppe $-CH(R^7)-CO-$ bedeutet.

4. Verfahren gemäss einem der Ansprüche 1-3, worin R 3-Nitrophenyl, 2-Chlor-5-nitrophenyl oder 2,5-Dichlor-

phenyl, $R^1$ Methyl, $R^2$ Wasserstoff und $R^3$ $C_1$-$C_4$-Alkyl, vorzugsweise Methyl, bedeuten.

5. Verfahren gemäss einem der Ansprüche 1-4, worin $R^4$ $C_1$-$C_6$-Alkyl, Cyan-$C_2$-$C_6$-alkyl, Halogen-$C_2$-$C_6$-alkyl, Hydroxy-$C_2$-$C_6$-alkyl, ω,ω,ω-Trifluor-$C_1$-$C_6$-alkyl, $C_1$-$C_6$-Alkoxy-$C_1$-$C_6$-alkyl, $C_1$-$C_4$-Alkanoyloxy-$C_1$-$C_6$-alkyl, Benzyloxy-$C_1$-$C_6$-alkyl oder gegebenenfalls durch Halogen substituiertes Phenyl-$C_1$-$C_6$-alkyl, vorzugsweise $C_1$-$C_6$-Alkyl, ω,ω,ω-Trifluor-$C_1$-$C_6$-alkyl, $C_1$-$C_6$-Alkoxy-$C_1$-$C_6$-alkyl oder Phenyl-$C_1$-$C_6$-alkyl, ganz besonders bevorzugt Isopropyl, 2,2,2-Trifluoräthyl, 2-Propoxyäthyl oder 1-Phenyläthyl, $R^5$ $C_1$-$C_6$-Alkyl, vorzugsweise Methyl, Aethyl oder Isopropyl, und $R^6$ und $R^7$ je Wasserstoff bedeuten.

6. Verfahren gemäss einem der Ansprüche 1-5, worin A die Gruppe -CH($R^7$)-CO-, R 3-Nitrophenyl, 2-Chlor-5-nitrophenyl oder 2,5-Dichlorphenyl, $R^1$ und $R^3$ je Methyl, $R^2$, $R^6$ und $R^7$ je Wasserstoff, $R^4$ Isopropyl, 2,2,2-Trifluoräthyl, 2-Propoxyäthyl oder 1-Phenyläthyl und $R^5$ Methyl, Aethyl oder Isopropyl bedeuten.

7. Verfahren gemäss Anspruch 1, dadurch gekennzeichnet, dass man 5-[(Aethylsulfamoyl)acetyl]-1,4-dihydro-2,6-dimethyl-4-(3-nitrophenyl)nicotinsäureisopropylester herstellt.

8. Verfahren gemäss Anspruch 1, dadurch gekennzeichnet, dass man 1,4-Dihydro-2,6-dimethyl-5-[(methylsulfamoyl)acetyl]-4-(3-nitrophenyl)nicotinsäureisopropylester herstellt.

9. Verfahren gemäss Anspruch 1, dadurch gekennzeichnet, dass man 1,4-Dihydro-2,6-dimethyl-5-[(methylsulfamoyl)acetyl]-4-(3-nitrophenyl)nicotinsäure-2,2,2--trifluoräthylester herstellt.

10. Verfahren gemäss Anspruch 1, dadurch gekennzeichnet, dass man 4-(2-Chlor-5-nitrophenyl)-1,4-dihydro-5-[(isopropylsulfamoyl)acetyl]-2,6-dimethylnicotinsäureisopropylester herstellt.

11. Verfahren gemäss Anspruch 1, dadurch gekennzeichnet, dass man 4-(2,5-Dichlorphenyl)-1,4-dihydro-5-[(isopropylsulfamoyl)acetyl]-2,6-dimethylnicotinsäureisopropylester herstellt.

12. Verfahren gemäss Anspruch 1, dadurch gekennzeichnet, dass man 1,4-Dihydro-5-[(isopropylsulfamoyl)-acetyl]-2,6-dimethyl-4-(3-nitrophenyl)nicotinsäure-2-propoxyäthylester herstellt.

13. Verfahren gemäss Anspruch 1, dadurch gekennzeichnet, dass man (S)-1,4-Dihydro-5-[(isopropylsulfamoyl)-acetyl]-2,6-dimethyl-4-(3-nitrophenyl)nicotinsäure-(S)-1-phenyläthylester herstellt.

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

EP 85107835.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 2 658 183 (BAYER)<br>* Ansprüche 1,4,5 *<br>-- | 1,15,16 | C 07 D 211/90<br>C 07 D 401/04<br>C 07 D 417/06<br>C 07 D 275/02<br>C 07 C 143/76<br>A 61 K 31/455 |
| A | DE - A1 - 2 747 513 (BAYER)<br>* Ansprüche 1,4,6 *<br>-- | 1,15,16 | |
| A | DE - A1 - 2 639 498 (BAYER)<br>* Ansprüche 1,4 *<br>-- | 1,15 | |
| A | EP - A1 - 0 063 767 (HOECHST)<br>* Zusammenfassung *<br>---- | 14 | |

RECHERCHIERTE SACHGEBIETE (Int. Cl.4)

C 07 D 211/00
C 07 D 401/00
C 07 D 417/00
C 07 D 275/00
C 07 C 143/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-23, 26
Unvollständig recherchierte Patentansprüche: 27 *)
Nicht recherchierte Patentansprüche: 24, 25
Grund für die Beschränkung der Recherche:

*)(soweit nicht die Anspr. 24,25 betreffend)
(Verfahren zur therapeutischen Behandlung des menschlichen oder tierischen Körpers (Artikel 52(4) EPÜ)

| Recherchenort | Abschlußdatum der Recherche | Prüfer |
|---|---|---|
| WIEN | 03-10-1985 | HOCHHAUSER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument